# EUROPEAN PATENT SPECIFICATION

(11) **EP 1 999 474 B1**
(45) Date of publication and mention of the grant of the patent: **26.10.2016**
(21) Application number: 07723746.9
(22) Date of filing: 29.03.2007
(51) Int. Cl.: G01N 33/68, C07K 16/00, C07K 16/44

(54) **METHOD FOR THE DETECTION AND/OR ENRICHMENT OF ANALYTE PROTEINS AND/OR ANALYTE PEPTIDES FROM A COMPLEX PROTEIN MIXTURE**
VERFAHREN ZUM NACHWEIS UND/ODER ZUR ANREICHERUNG VON ANALYTPROTEINEN UND/ODER -PEPTIDEN AUS EINEM KOMPLEXEN PROTEINGEMISCH
PROCÉDÉ POUR LA DÉTECTION ET/OU L'ENRICHISSEMENT DE PROTÉINES DE SUBSTANCES À ANALYSÉR ET/OU DE PEPTIDES DE SUBSTANCES À ANALYSER PROVENANT D'UN MÉLANGE COMPLEXE DE PROTÉINES

(30) Priority: 31.03.2006 DE 102006015001
(43) Date of publication of application: 10.12.2008
(73) Proprietor: NMI NATURWISSENSCHAFTLICHES UND MEDIZINISCHES INSTITUT AN DER UNIVERSITÄT TÜBINGEN, 72770 Reutlingen (DE)
(72) Inventor: STOLL, Dieter, 72116 Moessingen (DE); JOOS, Thomas, 72074 Tübingen (DE); TEMPLIN, Markus, 72074 Tuebingen (DE); POETZ, Oliver, 72076 Tuebingen (DE)
(74) Representative: Witte, Weller & Partner Patentanwälte mbB
(86) International application number: PCT/EP2007/002802
(87) International publication number: WO 2007/112927

(56) References cited:
- WO-A-02/37117
- WO-A-2004/031730
- WO-A-2004/081575
- US-A1- 2005 131 219
- SOLOVIEV M ET AL: "Peptidomics, current status" JOURNAL OF CHROMATOGRAPHY B: BIOMEDICAL SCIENCES & APPLICATIONS, ELSEVIER, AMSTERDAM, NL, vol. 815, no. 1-2, 5 February 2005 (2005-02-05), pages 11-24, XP004713815 ISSN: 1570-0232

### SEQUENCE LISTING

<110> NMI Naturwissenschaftliches und Medizinisches Institut an der Universität Tubingen
<120> Method for the detection and/or enrichment of analyte proteins from a complex protein mixture
<130> 3605P135WO
<150> DE 10 2006 015 001.5
   < 151 > 2006-03-31
<160> 99
<170> PatentIn version 3.1
<210> 1
   <211> 17
   <212> PRT
   <213> Artificial Sequence
<220>
   <223> c-myc
<400> 1
   His His His Gly Ser Gly Glu Gln Lys Leu Ile Ser Glu Glu Asp Leu
   1 5 10 15
   Gly
<210> 2
   <211> 16
   <212> PRT
   <213> Artificial Sequence
<220>
   <223> HA
<400> 2
   His His His Gly Ser Gly Tyr Pro Tyr Asp Val Pro Asp Tyr Ala Gly
   1 5 10 15
<210> 3
   <211> 18
   <212> PRT
   <213> Artificial Sequence
<220>
   <223> HAV
<400> 3
   His His His Gly Ser Gly Tyr Thr Asp Ile Glu Met Asn Arg Leu Gly
   1 5 10 15
   Lys Gly
<210> 4
   <211> 21
   <212> PRT
   <213> Artificial Sequence
<220>
   <223> V5
<400> 4
   His His His Gly Ser Gly Gly Lys Pro Ile Pro Asn Pro Leu Leu Gly
   1 5 10 15
   Leu Asp Ser Thr Gly
   20
<210> 5
   <211> 17
   <212> PRT
   <213> Artificial Sequence
<220>
   <223> c-myc
<400> 5
   Ser Glu Gln Lys Leu Ile Ser Glu Glu Asp Leu Gly Ser Gly His His
   1 5 10 15
   His
<210> 6
   <211> 16
   <212> PRT
   <213> Artificial Sequence
<220>
   <223> HA
<400> 6
   Ser Tyr Pro Tyr Asp Val Pro Asp Tyr Ala Gly Ser Gly His His His
   1 5 10 15
<210> 7
   <211> 18
   <212> PRT
   <213> Artificial Sequence
<220>
   <223> HAV
<400> 7
   Ser Tyr Thr Asp Ile Glu Met Asn Arg Leu Gly Lys Gly Ser Gly His
   1 5 10 15
   His His
<210> 8
   <211> 21
   <212> PRT
   <213> Artificial Sequence
<220>
   <223> V5
<400> 8
   Ser Gly Lys Pro Ile Pro Asn Pro Leu Leu Gly Leu Asp Ser Thr Gly
   1 5 10 15
   Ser Gly His His His
   20
<210> 9
   <211> 6
   <212> PRT
   <213> Artificial Sequence
<220>
   <223> Hexa-Histidin-Sequence
<400> 9
   His His His His His His
   1 5
<210> 10
   <211> 6
   <212> PRT
   <213> Artificial Sequence
<220>
   <223> Hexa-Histidine-Sequence
<220>
   <221> MOD_RES
   <222> (6)..(6)
   <223> AMIDATION
<400> 10
   His His His His His His
   1 5
<210> 11
   <211> 6
   <212> PRT
   <213> Artificial Sequence
<220>
   <223> Hexa-Histidine-Sequence
<220>
   <221> MISC_FEATURE
   <222> (1)..(1)
   <223> any amino acid
<400> 11
   Xaa His His His His His
   1 5
<210> 12
   <211> 6
   <212> PRT
   <213> Artificial Sequence
<220>
   <223> Hexa-Histidine-Sequence
<220>
   <221> MISC_FEATURE
   <222> (2)..(2)
   <223> any amino acid
<400> 12
   His Xaa His His His His
   1 5
<210> 13
   <211> 6
   <212> PRT
   <213> Artificial Sequence
<220>
   <223> Hexa-Histidine-Sequence
<220>
   <221> MISC_FEATURE
   <222> (3)..(3)
   <223> any amino acid
<400> 13
   His His Xaa His His His
   1 5
<210> 14
   <211> 6
   <212> PRT
   <213> Artificial Sequence
<220>
   <223> Hexa-Histidine-Sequence
<220>
   <221> MISC_FEATURE
   <222> (4)..(4)
   <223> any amino acid
<400> 14
   His His His Xaa His His
   1 5
<210> 15
   <211> 6
   <212> PRT
   <213> Artificial Sequence
<220>
   <223> Hexa-Histidine-Sequence
<220>
   <221> MISC_FEATURE
   <222> (5)..(5)
   <223> any amion acid
<400> 15
   His His His His Xaa His
   1 5
<210> 16
   <211> 6
   <212> PRT
   <213> Artificial Sequence
<220>
   <223> Hexa-Hsitidine-Sequence
<220>
   <221> MISC_FEATURE
   <222> (6)..(6)
   <223> any amino acid
<400> 16
   His His His His His Xaa
   1 5
<210> 17
   <211> 4
   <212> PRT
   <213> Homo sapiens
<400> 17
   Ala Gly Asp Arg
   1
<210> 18
   <211> 4
   <212> PRT
   <213> Homo sapiens
<400> 18
   Glu Ala Ser Arg
   1
<210> 19
   <211> 4
   <212> PRT
   <213> Homo sapiens
<400> 19
   Ile Val Ile Arg
   1
<210> 20
   <211> 4
   <212> PRT
   <213> Homo sapiens
<400> 20
   Met Ala Val Arg
   1
<210> 21
   <211> 4
   <212> PRT
   <213> Homo sapiens
<400> 21
   Thr Asn Val Lys
   1
<210> 22
   <211> 4
   <212> PRT
   <213> Homo sapiens
<400> 22
   Thr Val Leu Arg
   1
<210> 23
   <211> 5
   <212> PRT
   <213> Homo sapiens
<400> 23
   Asp Val His Asn Arg
   1 5
<210> 24
   <211> 5
   <212> PRT
   <213> Homo sapiens
<400> 24
   His Ala Ile Met Arg
   1 5
<210> 25
   <211> 5
   <212> PRT
   <213> Homo sapiens
<400> 25
   His Leu Thr Ser Arg
   1 5
<210> 26
   <211> 5
   <212> PRT
   <213> Homo sapiens
<400> 26
   Met Ser Glu Asp Lys
   1 5
<210> 27
   <211> 5
   <212> PRT
   <213> Homo sapiens
<400> 27
   Pro Gln Asp Tyr Lys
   1 5
<210> 28
   <211> 6
   <212> PRT
   <213> Homo sapiens
<400> 28
   Thr Tyr Thr Tyr Glu Lys
   1 5
<210> 29
   <211> 7
   <212> PRT
   <213> Artificial Sequence
<220>
   <223> peptide fragment of beta-catenin
<400> 29
   Ala His Gln Asp Thr Gln Arg
   1 5
<210> 30
   <211> 7
   <212> PRT
   <213> Homo sapiens
<400> 30
   Ala Ile Pro Glu Leu Thr Lys
   1 5
<210> 31
   <211> 7
   <212> PRT
   <213> Homo sapiens
<400> 31
   Ala Thr Val Gly Leu Ile Arg
   1 5
<210> 32
   <211> 7
   <212> PRT
   <213> Homo sapiens
<400> 32
   Glu Gln Gly Ala Ile Pro Arg
   1 5
<210> 33
   <211> 7
   <212> PRT
   <213> Homo sapiens
<400> 33
   Leu Ala Gly Gly Leu Gln Lys
   1 5
<210> 34
   <211> 7
   <212> PRT
   <213> Homo sapiens
<400> 34
   Leu Leu Trp Thr Thr Ser Arg
   1 5
<210> 35
   <211> 7
   <212> PRT
   <213> Homo sapiens
<400> 35
   Leu Val Gln Leu Leu Val Arg
   1 5
<210> 36
   <211> 7
   <212> PRT
   <213> Homo sapiens
<400> 36
   Met Val Ala Leu Leu Asn Lys
   1 5
<210> 37
   <211> 7
   <212> PRT
   <213> Homo sapiens
<400> 37
   Gln Asp Asp Pro Ser Tyr Arg
   1 5
<210> 38
   <211> 8
   <212> PRT
   <213> Homo sapiens
<400> 38
   Glu Gly Leu Leu Ala Ile Phe Lys
   1 5
<210> 39
   <211> 8
   <212> PRT
   <213> Artificial Sequence
<220>
   <223> peptide fragment of beta-catenin
<400> 39
   Asn Leu Ser Asp Ala Ala Thr Lys
   1 5
<210> 40
   <211> 9
   <212> PRT
   <213> Homo sapiens
<400> 40
   Leu Ala Glu Pro Ser Gln Met Leu Lys
   1 5
<210> 41
   <211> 9
   <212> PRT
   <213> Homo sapiens
<400> 41
   Ser Gly Gly Ile Pro Ala Leu Val Lys
   1 5
<210> 42
   <211> 9
   <212> PRT
   <213> Homo sapiens
<400> 42
   Val Leu Ser Val Cys Ser Ser Asn Lys
   1 5
<210> 43
   <211> 10
   <212> PRT
   <213> Homo sapiens
<400> 43
   Ala Ala Val Met Val His Gln Leu Ser Lys
   1 5 10
<210> 44
   <211> 10
   <212> PRT
   <213> Homo sapiens
<400> 44
   Leu His Tyr Gly Leu Pro Val Val Val Lys
   1 5 10
<210> 45
   <211> 10
   <212> PRT
   <213> Homo sapiens
<400> 45
   Leu Val Gln Asn Cys Leu Trp Thr Leu Arg
   1 5 10
<210> 46
   <211> 10
   <212> PRT
   <213> Homo sapiens
<400> 46
   Ser Pro Gln Met Val Ser Ala Ile Val Arg
   1 5 10
<210> 47
   <211> 11
   <212> PRT
   <213> Homo sapiens
<400> 47
   Leu Ser Val Glu Leu Thr Ser Ser Leu Phe Arg
   1 5 10
<210> 48
   <211> 12
   <212> PRT
   <213> Homo sapiens
<400> 48
   Glu Asp Ile Thr Glu Pro Ala Ile Cys Ala Leu Arg
   1 5 10
<210> 49
   <211> 12
   <212> PRT
   <213> Homo sapiens
<400> 49
   His Gln Glu Ala Glu Met Ala Gln Asn Ala Val Arg
   1 5 10
<210> 50
   <211> 12
   <212> PRT
   <213> Homo sapiens
<400> 50
   Leu Leu His Pro Pro Ser His Trp Pro Leu Ile Lys
   1 5 10
<210> 51
   <211> 12
   <212> PRT
   <213> Homo sapiens
<400> 51
   Leu Leu Asn Asp Glu Asp Gln Val Val Val Asn Lys
   1 5 10
<210> 52
   <211> 12
   <212> PRT
   <213> Homo sapiens
<400> 52
   Thr Met Gln Asn Thr Asn Asp Val Glu Thr Ala Arg
   1 5 10
<210> 53
   <211> 13
   <212> PRT
   <213> Homo sapiens
<400> 53
   Cys Thr Ala Gly Thr Leu His Asn Leu Ser His His Arg
   1 5 10
<210> 54
   <211> 13
   <212> PRT
   <213> Homo sapiens
<400> 54
   Asn Leu Ala Leu Cys Pro Ala Asn His Ala Pro Leu Arg
   1 5 10
<210> 55
   <211> 13
   <212> PRT
   <213> Homo sapiens
<400> 55
   Val Ala Ala Gly Val Leu Cys Glu Leu Ala Gln Asp Lys
   1 5 10
<210> 56
   <211> 14
   <212> PRT
   <213> Homo sapiens
<400> 56
   Met Met Val Cys Gln Val Gly Gly Ile Glu Ala Leu Val Arg
   1 5 10
<210> 57
   <211> 14
   <212> PRT
   <213> Homo sapiens
<400> 57
   Asn Glu Gly Val Ala Thr Tyr Ala Ala Ala Val Leu Phe Arg
   1 5 10
<210> 58
   <211> 15
   <212> PRT
   <213> Homo sapiens
<400> 58
   Thr Ser Met Gly Gly Thr Gln Gln Gln Phe Val Glu Gly Val Arg
   1 5 10 15
<210> 59
   <211> 17
   <212> PRT
   <213> Homo sapiens
<400> 59
   Leu Ile Ile Leu Ala Ser Gly Gly Pro Gln Ala Leu Val Asn Ile Met
   1 5 10 15
   Arg
<210> 60
   <211> 17
   <212> PRT
   <213> Homo sapiens
<400> 60
   Met Glu Glu Ile Val Glu Gly Cys Thr Gly Ala Leu His Ile Leu Ala
   1 5 10 15
   Arg
<210> 61
   <211> 18
   <212> PRT
   <213> Homo sapiens
<400> 61
   His Ala Val Val Asn Leu Ile Asn Tyr Gln Asp Asp Ala Glu Leu Ala
   1 5 10 15
   Thr Arg
<210> 62
   <211> 18
   <212> PRT
   <213> Homo sapiens
<400> 62
   Met Ala Thr Gln Ala Asp Leu Met Glu Leu Asp Met Ala Met Glu Pro
   1 5 10 15
   Asp Arg
<210> 63
   <211> 20
   <212> PRT
   <213> Homo sapiens
<400> 63
   Phe Leu Ala Ile Thr Thr Asp Cys Leu Gln Ile Leu Ala Tyr Gly Asn
   1 5 10 15
   Gln Glu Ser Lys
   20
<210> 64
   <211> 21
   <212> PRT
   <213> Homo sapiens
<400> 64
   Gly Leu Asn Thr Ile Pro Leu Phe Val Gln Leu Leu Tyr Ser Pro Ile
   1 5 10 15
   Glu Asn Ile Gln Arg
   20
<210> 65
   <211> 22
   <212> PRT
   <213> Homo sapiens
<400> 65
   Glu Ala Ala Glu Ala Ile Glu Ala Glu Gly Ala Thr Ala Pro Leu Thr
   1 5 10 15
   Glu Leu Leu His Ser Arg
   20
<210> 66
   <211> 22
   <212> PRT
   <213> Homo sapiens
<400> 66
   Pro Ala Ile Val Glu Ala Gly Gly Met Gln Ala Leu Gly Leu His Leu
   1 5 10 15
   Thr Asp Pro Ser Gln Arg
   20
<210> 67
   <211> 26
   <212> PRT
   <213> Artificial Sequence
<220>
   <223> peptide fragment of beta-catenin
<400> 67
   Thr Glu Pro Met Ala Trp Asn Glu Thr Ala Asp Leu Gly Leu Asp Ile
   1 5 10 15
   Gly Ala Gln Gly Glu Pro Leu Gly Tyr Arg
   20 25
<210> 68
   <211> 28
   <212> PRT
   <213> Artificial Sequence
<220>
   <223> peptide fragment of beta-catenin
<400> 68
   Met Leu Gly Ser Pro Val Asp Ser Val Leu Phe Tyr Ala Ile Thr Thr
   1 5 10 15
   Leu His Asn Leu Leu Leu His Gln Glu Gly Ala Lys
   20 25
<210> 69
   <211> 29
   <212> PRT
   <213> Homo sapiens
<400> 69
   Ala Ala Met Phe Pro Glu Thr Leu Asp Glu Gly Met Gln Ile Pro Ser
   1 5 10 15
   Thr Gln Phe Asp Ala Ala His Pro Thr Asn Val Gln Arg
   20 25
<210> 70
   <211> 30
   <212> PRT
   <213> Homo sapiens
<400> 70
   Ala Ala Val Ser His Trp Gln Gln Gln Ser Tyr Leu Asp Ser Gly Ile
   1 5 10 15
   His Ser Gly Ala Thr Thr Thr Ala Pro Ser Leu Ser Gly Lys
   20 25 30
<210> 71
   <211> 39
   <212> PRT
   <213> Homo sapiens
<400> 71
   Gln Glu Gly Met Glu Gly Leu Leu Gly Thr Leu Val Gln Leu Leu Gly
   1 5 10 15
   Ser Asp Asp Ile Asn Val Val Thr Cys Ala Ala Gly Ile Leu Ser Asn
   20 25 30
   Leu Thr Cys Asn Asn Tyr Lys
   35
<210> 72
   <211> 41
   <212> PRT
   <213> Homo sapiens
<400> 72
   Gly Asn Pro Glu Glu Glu Asp Val Asp Thr Ser Gln Val Leu Tyr Glu
   1 5 10 15
   Trp Glu Gln Gly Phe Ser Gln Ser Phe Thr Gln Glu Gln Val Ala Asp
   20 25 30
   Ile Asp Gly Gln Tyr Ala Met Thr Arg
   35 40
<210> 73
   <211> 64
   <212> PRT
   <213> Homo sapiens
<400> 73
   Ser Phe His Ser Gly Gly Tyr Gly Gln Asp Ala Leu Gly Met Asp Pro
   1 5 10 15
   Met Met Glu His Glu Met Gly Gly His His Pro Gly Ala Asp Tyr Pro
   20 25 30
   Val Asp Gly Leu Pro Asp Leu Gly His Ala Gln Asp Leu Met Asp Gly
   35 40 45
   Leu Pro Pro Gly Asp Ser Asn Gln Leu Ala Trp Phe Asp Thr Asp Leu
   50 55 60
<210> 74
   <211> 84
   <212> PRT
   <213> Homo sapiens
<400> 74
   Gly Asn Pro Glu Glu Glu Asp Val Asp Thr Ser Gln Val Leu Tyr Glu
   1 5 10 15
   Trp Glu Gln Gly Phe Ser Gln Ser Phe Thr Gln Glu Gln Val Ala Asp
   20 25 30
   Ile Asp Gly Gln Tyr Ala Met Thr Arg Ala Gln Arg Val Arg Ala Ala
   35 40 45
   Met Phe Pro Glu Thr Leu Asp Glu Gly Met Gln Ile Pro Ser Thr Gln
   50 55 60
   Phe Asp Ala Ala His Pro Thr Asn Val Gln Arg Leu Ala Glu Pro Ser
   65 70 75 80
   Gln Met Leu Lys
<210> 75
   <211> 4
   <212> PRT
   <213> Artificial Sequence
<220>
   <223> c-terminus of beta catenin peptide fragment
<400> 75
   Leu Ser Gly Lys
   1
<210> 76
   <211> 4
   <212> PRT
   <213> Artificial Sequence
<220>
   <223> c-terminus of beta catenin peptide fragment
<400> 76
   Ala Met Thr Arg
   1
<210> 77
   <211> 4
   <212> PRT
   <213> Artificial Sequence
<220>
   <223> c-terminus of beta catenin peptide fragment
<400> 77
   Asp Thr Asp Leu
   1
<210> 78
   <211> 4
   <212> PRT
   <213> Artificial Sequence
<220>
   <223> c-terminus of beta catenin peptide fragment
<400> 78
   Leu Gly Tyr Arg
   1
<210> 79
   <211> 4
   <212> PRT
   <213> Artificial Sequence
<220>
   <223> c-terminus of beta catenin peptide fragment
<400> 79
   Gln Met Leu Lys
   1
<210> 80
   <211> 8
   <212> PRT
   <213> Artificial Sequence
<220>
   <223> c- and n-terminus of beta catenin peptide fragment combined
<220>
   <221> MISC_FEATURE
   <222> (4)..(4)
   <223> any amino acid, 1 to 100
<400> 80
   Ala Ala Val Xaa Leu Ser Gly Lys
   1 5
<210> 81
   <211> 8
   <212> PRT
   <213> Artificial Sequence
<220>
   <223> c- and n-terminus of beta catenin peptide fragment combined
<220>
   <221> MISC_FEATURE
   <222> (4)..(4)
   <223> any amino acid, 1 to 100
<400> 81
   Gly Asn Pro Xaa Ala Met Thr Arg
   1 5
<210> 82
   <211> 8
   <212> PRT
   <213> Artificial Sequence
<220>
   <223> c- and n-terminus of beta catenin peptide fragment combined
<220>
   <221> MISC_FEATURE
   <222> (4)..(4)
   <223> any amino aicd, 1 to 100
<400> 82
   Ser Phe His Xaa Asp Thr Asp Leu
   1 5
<210> 83
   <211> 8
   <212> PRT
   <213> Artificial Sequence
<220>
   <223> c- and n-terminus of beta catenin peptide fragment combined
<220>
   <221> MISC_FEATURE
   <222> (4)..(4)
   <223> any amino acid, 1 to 100
<400> 83
   Thr Glu Pro Xaa Leu Gly Tyr Arg
   1 5
<210> 84
   <211> 8
   <212> PRT
   <213> Artificial Sequence
<220>
   <223> c- and n-terminus of beta catenin peptide fragment combined
<220>
   <221> MISC_FEATURE
   <222> (4)..(4)
   <223> any amino acid, 1 to 100
<400> 84
   Gly Asn Pro Xaa Gln Met Leu Lys
   1 5
<210> 85
   <211> 4
   <212> PRT
   <213> Artificial Sequence
<220>
   <223> C-terminus of a beta-catenin peptide fragment
<400> 85
   Ala Pro Phe Lys
   1
<210> 86
   <211> 8
   <212> PRT
   <213> Artificial Sequence
<220>
   <223> C-terminus of a beta-catenin peptide-fragment modified with space
   r
<220>
   <221> MISC_FEATURE
   <222> (2)..(4)
   <223> three spacer amino acids each being 3,6-dioxa-8-aminooctanoic aci
   d
<400> 86
   Cys Xaa Xaa Xaa Ala Met Thr Arg
   1 5
<210> 87
   <211> 15
   <212> PRT
   <213> Artificial Sequence
<220>
   <223> C-terminus of a beta-catenin peptide fragment fused to myc
<400> 87
   Cys Glu Gln Lys Leu Ile Ser Glu Glu Asp Leu Ala Met Thr Arg
   1 5 10 15
<210> 88
   <211> 14
   <212> PRT
   <213> Artificial Sequence
<220>
   <223> C-terminus of a beta-catenin peptide fragment fuse to ha
<400> 88
   Cys Tyr Pro Tyr Asp Val Pro Asp Tyr Ala Ala Met Thr Arg
   1 5 10
<210> 89
   <211> 15
   <212> PRT
   <213> Artificial Sequence
<220>
   <223> C-terminus of a beta-catenin peptide fragment fused to myc with c
   -terminus blocked
<400> 89
   Cys Glu Gln Lys Leu Ile Ser Glu Glu Asp Leu Ala Met Thr Arg
   1 5 10 15
<210> 90
   <211> 14
   <212> PRT
   <213> Artificial Sequence
<220>
   <223> C-terminus of a beta-catenin peptide fragment fused to ha with c-terminus blocked
<400> 90
   Cys Tyr Pro Tyr Asp Val Pro Asp Tyr Ala Ala Met Thr Arg
   1 5 10
<210> 91
   <211> 10
   <212> PRT
   <213> Artificial Sequence
<220>
   <223> C-terminus of a beta-catenin peptide fragment randomized
<220>
   <221> MISC_FEATURE
   <222> (10)..(10)
   <223> any amino acid
<400> 91
   Cys Ser Glu Glu Asp Leu Ala Met Thr Xaa
   1 5 10
<210> 92
   <211> 10
   <212> PRT
   <213> Artificial Sequence
<220>
   <223> C-terminus of a beta-catenin peptide fragment randomized
<220>
   <221> MISC_FEATURE
   <222> (9)..(9)
   <223> any amino acid
<400> 92
   Cys Ser Glu Glu Asp Leu Ala Met Xaa Arg
   1 5 10
<210> 93
   <211> 10
   <212> PRT
   <213> Artificial Sequence
<220>
   <223> C-terminus of a beta-catenin peptide fragment randomized
<220>
   <221> MISC_FEATURE
   <222> (8)..(8)
   <223> any amino acid
<400> 93
   Cys Ser Glu Glu Asp Leu Ala Xaa Met Arg
   1 5 10
<210> 94
   <211> 10
   <212> PRT
   <213> Artificial Sequence
<220>
   <223> c-terminus of a beta catenin peptide fragment randomized
<220>
   <221> MISC_FEATURE
   <222> (7)..(7)
   <223> any amino acid
<400> 94
   Cys Ser Glu Glu Asp Leu Xaa Met Thr Arg
   1 5 10
<210> 95
   <211> 9
   <212> PRT
   <213> Artificial Sequence
<220>
   <223> modified c-terminus of a beta-catenin peptide fragment
<220>
   <221> MISC_FEATURE
   <222> (1)..(1)
   <223> Dinitrophenyl-modified
<400> 95
   Asp Gly Gly Gln Tyr Ala Met Thr Arg
   1 5
<210> 96
   <211> 13
   <212> PRT
   <213> Artificial Sequence
<220>
   <223> c myc plus histidine tag
<220>
   <221> MISC_FEATURE
   <222> (1)..(1)
   <223> Dinitrophenyl modified
<400> 96
   Glu Gln Lys Leu Ile Ser Glu Glu Asp Leu His His His
   1 5 10
<210> 97
   <211> 16
   <212> PRT
   <213> Artificial Sequence
<220>
   <223> c myc plus spacer plus histidine tag
<220>
   <221> MISC_FEATURE
   <222> (11)..(13)
   <223> 3,6-Dooxa-8-aminooctanoic acid
<220>
   <221> MISC_FEATURE
   <222> (11)..(1)
   <223> Dinitrophenyl-modified
<400> 97
   Glu Gln Lys Leu Ile Ser Glu Glu Asp Leu Xaa Xaa Xaa His His His
   1 5 10 15
<210> 98
   <211> 15
   <212> PRT
   <213> Artificial Sequence
<220>
   <223> HA plus spacer plus histidine tag
<220>
   <221> MISC_FEATURE
   <222> (10)..(12)
   <223> 3,6-Dioxa-8-aminoocatnoic acid
<220>
   <221> MISC_FEATURE
   <222> (1)..(1)
   <223> Dinitrophenyl-modified
<400> 98
   Tyr Pro Tyr Asp Val Pro Asp Tyr Ala Xaa Xaa Xaa His His His
   1 5 10 15
<210> 99
   <211> 17
   <212> PRT
   <213> Artificial Sequence
<220>
   <223> HAV plus spacer plus histidine tag
<220>
   <221> MISC_FEATURE
   <222> (12)..(14)
   <223> 3,6-Dioxa-8-aminooctanoic acid
<220>
   <221> MISC_FEATURE
   <222> (1)..(1)
   <223> Dinitrophenyl- modified
<400> 99
   Tyr Thr Asp Ile Glu Met Asn Arg Leu Gly Lys Xaa Xaa Xaa His His
   1 5 10 15
   His

## Description

The present invention relates to a method for the detection and/or enrichment of various analyte proteins and/or analyte peptides from a protein mixture which includes proteins and/or peptides.

Binding molecules are disclosed which are specific for the terminus of various peptide analytes, and to the use thereof in a method for the detection and/or enrichment of various analyte proteins and/or analyte peptides from a protein mixture.

Methods and binding molecules of these types are extensively known in the prior art.

The areas of use of the known methods are for example protein analysis and protein detection in complex samples, especially methods for analysis of the proteome in general.

By "proteome" is meant the quantitative totality of the proteins in a cell, a tissue or an organism, i.e. the knowledge of all the expressed proteins in all isoforms, poly-morphisms and post-translational modification, and their respective concentrations, in particular at a defined time and under defined external conditions.

Accordingly, the condition of a cell, of a tissue or of an organism is described in particular by the quantitative profile of its proteins. It may thus be for example that in a disease there is a reduction in the expression of certain proteins and an increase in the expression of other proteins, or that certain proteins are only then expressed at all, or that certain post-translational protein modifications are altered. The protein profile is therefore suitable as direct indicator of the respective current condition of cells, tissues, organs or organisms and thus as indicator of disease or health.

It is additionally possible to follow the effect of drugs and to estimate their side effects via changes in the protein profile.

In contrast to the mRNA profiles also frequently used for this purpose, the advantage of determining the protein profile is that a direct conclusion about the mechanisms involved is possible through the changing protein profiles, because cellular processes usually proceed with direct involvement of proteins, by which the functions of the cell are carried out.

For qualitative and quantitative detection of protein profiles it is necessary for methods for detecting proteins to detect most of the proteins even in complex samples, and it ought to be possible to detect quantitatively the amount of the proteins over a dynamic range which is as large as possible.

One difficulty associated with this is that the concentrations of the different proteins in most natural samples may differ by 9-12 orders of magnitude. In addition, there are no possibilities for amplification of proteins, in contrast to DNA or RNA.

Furthermore, there is as yet no method with which all proteins, i.e. both very acidic, very basic, very large, very small, hydrophobic and hydrophilic proteins, can be detected.

At present, two methods are used in principle for detecting complex proteomes:
- 2D electrophoresis with electrophoretic separation of different proteins in two dimensions and subsequent defined proteolysis of the separated proteins, and identification of the respective protein species via peptide masses by mass spectrometry.
- one-dimensional or multidimensional chromatographic separation of peptides from a defined proteolytic degradation of all proteins of a proteome with subsequent identification of the peptides by tandem mass spectrometry and bioinformatic assignment of the peptide fragments to the original proteins of the proteome via protein or genome databases.

These methods are supplemented by antibody-based methods in which proteins are detected qualitatively and quantitatively through the specific binding to corresponding antibodies. Examples thereof are Western blots, ELISA or antibody microarrays.

Two-dimensional gel electrophoresis (2D.PAGE) separates proteins in the first dimension according to isoelectric point and in the second dimension according to a molecular weight. It allows complex protein mixtures to be analysed with very high separation efficiency for up to 10 000 proteins. One disadvantage of this method is that only some of the proteins from the sample to be investigated are detected; very large and very small proteins, and very basic and very acidic protein species are not detected under standard conditions.

In addition, this method is time-consuming and difficult to reproduce. Because of the difference in staining efficiency of different proteins and of the small dynamic detection range, quantitative analyses are difficult and are possible only with great effort and sample consumption. It is moreover frequently possible to analyse only a relative small amount of protein per sample, and thus proteins which are present only in small amount in the sample are no longer detectable or at least no longer unambiguously detectable.

Chromatographic protein separation usually takes place according to molecular size (size exclusion chromatography), molecular charge (ion exchange chromatography) or hydrophobicity (reverse phase chromatography, hydrophobic interaction chromatography). The separation efficiency of the chromatographic methods is less than that of 2D-PAGE for proteins. For this reason, frequently very elaborate multidimensional separations are carried out for proteome analyses. Only antibody-based detection methods are currently able to identify individual analyte proteins from complex protein mixtures. However, established methods such as ELISA are unsuitable for analysing many analytes from one sample. Methods based on antibody arrays are at present limited in terms of the parallel detection of many different proteins from complex mixtures in particular by the small number of suitable highly selective antibodies.

Owing to the limitations of all currently available methods for proteome analysis in terms of parallel, rapid, reproducible and sensitive detection of analyte proteins from complex samples, reproducible fractionation of protein samples is a crucial operation for proteome analysis.

Conrads et al., "Cancer Proteomics: many technologies, one goal", Expert Rev. Proteomics 2(5), (2005), pp. 693-703, emphasise that it is important to identify from the enormous quantity of data obtained by methods of proteome analysis the bi-omarkers which are specific for cancer or other diseases.

WO 2004/031730 discloses a flow-through method for determining amount of target protein in a sample. A specific binding reagent like an antibody is used to capture and thus enrich a specific monitor peptide in a proteolytic digest of a complex protein sample and an internal standard peptide having the same chemical structure as the monitor peptide but being labeled. Upon elution into a mass spectrometer both peptides are quantitated.

The specific binding agent or antibody shall reversely bind a specific peptide sequence of about 5 to 20 amino acid residues in order to be able to capture specific peptides from a mixture of peptides arising from the specific cleavage of e.g. human serum by proteolytic enzymes.

The monitor peptide has to be highly specific for the target, i.e. should not share homology with any other protein of the target organism.

By this, it shall be possible to enrich one specific peptide using e.g. first an N-terminal antibody, and in a sequential second enrichment step a C-terminal antibody.

Further, this document describes the sequential use of two antibodies, each highly specific for a peptide, in order to enhance the specificity and sensitivity of the method disclosed.

Document US 2005/0131219 A1 discloses the use of a small epitope specific antibody recognizing a multiplicity of proteins that comprise the small epitope, to reduce the complexity of a sample.

In view of the aspects of proteome analysis described in the literature and mentioned above and of the possibilities associated therewith for developing novel diagnostic methods, active ingredients and therapies, novel technologies which avoid the disadvantages of known analytical methods are of enormous importance.

It is therefore an object of the present invention to provide a novel method or a tool with whose aid proteomes or subproteomes can be analysed qualitatively and/or quantitatively.

In the method mentioned at the outset, this object is achieved according to the invention by the steps:
a) provision of the sample mixture and, fragmentation of the proteins contained therein into defined peptides by tryptic digestion,
b) provision of first binding molecules which are specific for a peptide epitope of a large number of the various analyte proteins and/or analyte peptides, whereby the peptide epitope includes three, four or five amino acids,
c) incubation of the first binding molecules with the sample mixture, and
d) detection and/or enrichment of the various analyte proteins and/or analyte peptides bound to the first binding molecules, by using second binding molecules which are specific for a peptide epitope of a large number of the various analyte proteins and/or analyte peptides, whereby the peptide epitope includes three, four or five amino acids, and specifically recognize analyte proteins and/or analyte peptides which are bound to the first binding molecules, wherein
   the first binding molecules are specific for one of the two terminal peptide epitopes of the analyte peptides, this terminal peptide epitope including the free NH₂ group or the free COOH group and 3 to 5 amino acids, and
   the second binding molecules are specific for the other terminal peptide epitope of the analyte peptides, whereby the other terminal peptide epitope includes the free NH₂ group or the free COOH group and 3 to 5 amino acids,
   such that an analyte peptide is detected by the combined binding of first and second binding molecules to both free terminal epitopes of the analyte peptide.

The invention is based on the surprising realization by the inventors that the binding molecules employed according to the invention can be utilized for the detection and/or enrichment of various analyte proteins and/or analyte peptides even from a complex sample mixture, although because of the recognition sequence with defined amino acids in only five, preferably four, or three positions, they bind to a large number of analyte proteins or analyte peptides, that is to say are rather unselective.

Individual positions in the recognition sequence may moreover even be occupied only by partly defined amino acids, that is to say ones belonging to a group of amino acids. An example of the distribution of defined and partly defined amino acids in such a recognition sequence would be OOXXO, OOXXX, or OXOXO, where O represents the defined amino acids and X represents a group of amino acids such as, for example, the group of hydrophobic, aliphatic or aromatic amino acids.

Thus, the binding molecules employed according to the invention specifically recognize epitopes having up to a maximum of five amino acids.

"Binding molecule" means herein any molecule or any substance which is able to bind to a peptide/protein or to which a peptide/protein can bind.

It is understood in the context of the present invention that it is possible to employ in this case any binding molecule which specifically recognizes a peptide epitope having up to 5 amino acids.

"Analyte proteins/peptides" mean in the context of the present invention those proteins/peptides which bind from a complex sample mixture/protein mixture to the binding molecules in step c).

Since the binding molecules to be employed in the method according to the invention recognize peptide epitopes which have up to a maximum of five amino acids, the probability that a large number of peptides/proteins has this epitope is high, so that there is also binding of a large number of peptides/proteins by a particular binding molecule in each case. Binding molecules which bind to different analyte proteins or peptides are categorized in the prior art as unsuitable for use in biological/biochemical studies.

It is understood in the context of the present invention that more peptides/proteins of a proteome having a particular epitope means fewer amino acids in the corresponding epitope specifically recognized by the binding molecule and more different amino acids permitted per position.

This means that on use of binding molecules which are specific for epitopes having, for example, only 3 amino acid residues, far more peptides/proteins are bound than on use of binding molecules which specifically recognize an epitope having, for example, five amino acid residues.

It is further understood that in the context of the present invention it is also possible to provide in step b) two or more different first binding molecules, so that the amount of analyte proteins/peptides to be bound is increased further. From this emerges the surprising possibility of binding all the proteins/peptides of a proteome with a limited number of binding molecules which can be prepared according to the current state of the art. With the 20 proteinogenic amino acids, the number of theoretically necessary different binding molecules which specifically bind 3 defined amino acids is 20³ = 8000. By contrast, in the case of binding via epitopes having 5 defined amino acids, as many as 20⁵ = 3.2 million different binding molecules would be necessary in order to bind all theoretically possible epitopes of a protein.

Thus, contrary to the method described in WO 2004/031730 mentioned at the outset, the inventive concept resides in using two per se unspecific binding molecules that bind simultaneously to the analyte protein or analyte peptide and by this enable a specific enrichment and/or determination of the analyte protein/peptide.

A further advantage of the method according to the invention is that with the 2 × 20³ binding molecules it is possible to detect all N/C termini theoretically conceivable for proteinogenic amino acids of all peptides of any proteomes, independent of species.

It is understood that the selection of the amino acid triplets and thus of the amino acids depends per se on the sample to be investigated. Thus, depending on the sample to be investigated, account must also be taken of modified amino acids, or else - in the case of non-human samples - amino acids which are to be found only in animal, plant or microbial samples. It would be clear to the skilled person in this connection - based on the prior art available concerning this - which amino acids must be taken into account for which sample analysis.

The approach according to the invention thus makes possible for the first time an array-based proteome analysis.

In this method, therefore, there is initial provision of a complex sample mixture/protein mixture which includes proteins and/or peptides, it being possible for this sample mixture to be any sample/protein mixture obtained from any tissue or a fluid, such as, for example, a tissue homogenate, serum, etc. This protein mixture can be additionally denatured by adding denaturing agents such as, for example, urea or guanidinium hydrochloride, and by reduction and subsequent alkylation, so that preferably unfolded protein chains which are accessible for proteases are present. The native or denatured sample/protein mixture is treated with selectively cleaving protease trypsin, which cleave the peptides/proteins present in the sample into smaller fragments which are defined by the specificity of the protease. There also exist a whole series of endo- or exoproteases which are known to the skilled person and can be employed for specific proteolysis. It is possible through the choice of the denaturation of the protein sample to control the number of possible peptides cleaved in the proteolysis and to adjust the complexity of the analytical sample. Further chemical fragmentation can be applied, e.g. with cyanogen bromide (C side of Met).

Digestion with one or more proteases and/or chemical fragmentation results in a peptide mixture which is provided in step a). After provision of the binding molecules in step b), the peptide mixture is incubated with the binding molecules, during which the binding molecules bind to the appropriate epitopes of the peptides, and the corresponding analyte peptides bound to the binding molecules can be detected/enriched.

This method has the advantage that the proteolytic degradation makes the termini of the individual analyte peptides available for the binding molecules.

The binding molecules employed according the invention bind directly to the C- or N-terminal end, thus making it possible to greatly reduce the cross-reactivity even further with short binding epitopes. At the same time, the proteolytic fragmentation of an analyte protein generates a plurality of detectable analyte proteins, making redundant detection possible.

According to the invention, first binding molecules which are specific for one of the two terminal peptide epitopes of the analyte proteins and/or analyte peptides are provided in step b), whereby the terminal peptide epitope includes the free NH₂ group or the free COOH group and in each case up to a maximum of five amino acids.

The advantage is that effective tools which bind specifically and stably to the respective termini are provided with the binding molecules to be employed according to the invention. A contribution is made to this by the realization by the inventors that only up to a maximum of five amino acids are necessary for stable binding, and that the terminal functional group may have such a strong influence on the binding that, in the case of terminal epitopes having few amino acids, no cross-reactivity to internal epitopes having the same amino acid sequence occurs. The binding to the termini of the peptides additionally results in the possibility of being able in a subsequent step to employ a further binding molecule which can bind to the other terminal peptide epitope of the isolated/identified analyte peptide, so that further selection of the peptides is possible by use of a further binding molecule.

According to the invention the second binding molecules are specific for the respectively other terminal peptide epitope, the respective other peptide epitope including the free NH₂ group or the COOH group and in each case up to a maximum of five amino acids.

This has the advantage that, out of the large number of different analyte peptides which have bound to the first binding molecule, only certain analyte peptides are bound by the second binding molecules, in particular precisely those whose other terminal peptide epitope is specifically recognized by the second binding molecules. It is thus possible for the analyte proteins/peptides to be further grouped or selected in a targeted manner. The specificity of the corresponding second binding molecule can be used for targeted further restriction of the amount of the analyte proteins/analyte peptides bound by the first binding molecules, i.e. a more specific second binding molecule will bind a smaller amount of the peptides, and vice versa.

Through the combined binding of binding molecules to two short terminal epitopes (C terminus and N terminus), which each consist of a maximum of five amino acids, it is surprisingly possible to detect the peptide specifically even if the binding of each individual binding molecule itself also occurs with a relatively large number of different peptides of a proteome. The described method accordingly makes it possible to detect a particular peptide unambiguously through a split specific epitope.

The object underlying the invention is completely achieved in this way.

In a preferred embodiment, a sample mixture with denatured analyte proteins and/or analyte peptides is employed in step a).

This embodiment has the advantage that on use of denatured proteins the proteins which are in denatured form in the sample mixture are more easily accessible for the at least one binding molecule and can bind the latter better.

In a further embodiment, first and/or second binding molecules which display amino acid group-specific recognition at one or more positions of the peptide epitope are provided in step b).

This embodiment then has the advantage that binding molecules which are specific for an epitope having a maximum of up to five amino acids are provided, with at least one of these amino acids being recognized merely group-specifically, that is to say for example on the basis of a positive or negative charge of the relevant amino acid, because of the hydrophobically aliphatic property of the amino acid, etc. It is known in the state of the art to classify amino acids into groups having similar/identical properties. Thus, for example, the aliphatic hydrophobic amino acids include alanine, valine, leucine and isoleucine, the aromatic amino acids include tryptophan, tyrosine and phenylalanine, the acidic amino acids include aspartic acid and glutamic acid, and the basic amino acids include lysine, histidine and arginine. It is therefore possible in accordance with such group classifications to generate and provide binding molecules which for example within the appropriate epitope recognize at its position 3 apart from glycine also alanine, valine, leucine and isoleucine, and thus overall bind more peptides than binding molecules which do not display group-specific recognition for at least one position of the peptide.

It is preferred in one embodiment of the method according to the invention for the at least first binding molecules to be immobilized on a support. It is particularly preferred in this connection for the support to be selected from the group including microarrays, support material for affinity columns, chromatography materials, microchannel structures, capillary surfaces, sensor surfaces, polymeric porous sponge structures, microspheres (or beads).

This embodiment has the advantage that the binding molecules can be more easily manipulated and provided in step b), through their immobilization on a support, and thus overall represent a practical tool for the method according to the invention. It is possible in this connection for the binding molecules to be applied for example exactly defined in an array on the support, in relation both to the amount of binding molecules and to the alignment and arrangement on the support, these parameters depending on the support material to be employed in each case. It is then advantageously possible for the support and the analyte peptides bound via the binding molecules to the support to be further analyzed.

Suitable examples of beads (or "microspheres") in the present case are coded beads (for example fluorescence- or colour-coded) or magnetic beads, inter alia, and it will be clear to the skilled person which beads are suitable for the particular use.

It is further possible to provide in the context of the present invention for the same binding molecule to be present in each case on the individual beads, or else for different binding molecules to be present on one bead, so that many different analyte peptides are removed from the sample mixture by binding with each individual bead.

Immobilization of the binding molecules on the supports can take place by methods known in the state of the art (see, for example, review by Stoll et al., FBS 2000, Hermanson, Greg. T., Bioconjugate Techniques, Academic Press).

It is preferred in this connection for the detection and/or enrichment in step d) to take place by simultaneous binding of first and second binding molecules to different epitopes of the analyte protein and/or analyte peptide by methods such as FRET, proximity ligation assay, etc., it further being preferred for the first and second binding molecules to be incubated in solution with the sample.

It is advantageous in this embodiment of the method according to the invention that both binding molecules bind their analyte protein/peptide in liquid phase. It is particularly preferred in this connection for the two binding molecules to be modified by labels such as, for example, dye pairs (fluorophore/quencher or fluorophore 1/fluorophore 2) or oligonucleotides which are appropriate for detection of the pairwise binding to the analyte molecule by means of various assays complying with the state of the art, such as fluorescence transfer (FRET) assays or proximity ligation assays; concerning this, see Gustafsdottir et al., Proximity ligation assays for sensitive and specific protein analyses, in Anal Biochem. 2005 Oct 1; 345(1): 2-9. Epub 2005 Feb 7, and Arai et al., Fluorolabeling of antibody variable domains with green fluorescent protein variants: application to an energy transfer-based homogeneous immunoassay, in Protein Eng. 2000 May; 13 (5): 369-76.

The inventors of the present application have realised that through the use of two binding molecules with, for example, in each case a three-amino acid specificity it is possible to attain at least the specificity of a binding molecule specific for six amino acids, because further parameters besides the three amino acids influence the specificity. In combination with the fact that in this case the C- and N-terminal end of each peptide fragment is recognized, specific detection of analyte peptides/proteins is possible through the combination of two short epitopes. The specificity/selectivity for the analyte peptides/proteins thus arises through the combined use of the two binding molecules, because a large number of analyte peptides/proteins is - deliberately - bound by the first binding molecule, and only with the use of the second binding molecule is the "overall specificity" significantly increased and reaches a level at least equal to that of a six amino acids-specific binding molecule.

The "dividing up" of a sextuplet epitope into two triplet epitopes - preferably one for the C-terminal and one for the N-terminal end - in this case leads to a drastic reduction in the binding molecules necessary for the analysis of all possible peptides. In the present case, only 2 × 20³ - instead of 20⁶ for a sextuplet epitope - different binding molecules are required in order to be able to detect all possible peptides. Accordingly, in the claimed approach only 2 × 8000 antibodies are necessary, that is to say in each case 8000 for the N-terminal and 8000 for the C-terminal end of the peptides. It is thus possible by providing a library of 2 × 8000 binding molecules to detect any desired analyte peptide from a peptide mixture. By comparison therewith, with a sextuplet epitope more than 10⁷ binding molecules would be necessary - for an identical analysis.

It is generally preferred in the method according to the invention for the first and the second binding molecules to be selected from the group including antibodies, antibody fragments, aptamers, recombinant binding molecules.

It is preferred in this connection for preparing the binding molecules to provide in a first step peptides having C- and N-terminal triplets, with the amino acid triplets displaying all possible amino acid combinations which are possible starting from 20 proteinogenic amino acids, and in the subsequent step to employ these peptides for immunization, selection and affinity maturation methods.

It is possible in this connection to employ for example classical immunization methods which are sufficiently well described in the state of the art (see, for example, Antibodies: A Laboratory Manual, by Ed Harlow, David Lane). On the other hand, the binding molecules can also be generated *in vitro,* in which case the binding pocket is constructed in such a way that the terminal NH₃⁺/COO⁻ function can bind optimally, for example in the form of a recess with appropriate opposite charge.

The methods employed for the synthetic preparation of the peptides employed for the immunization are likewise sufficiently well known in the state of the art (see, for example, Fmoc Solid Phase Peptide Synthesis, A Practical Approach by W.C. Chan and P.D. White (Eds), Oxford University Press).

It is understood that the features and advantages mentioned above and to be explained hereinafter can be used not only in the stated combination but also alone or in other combinations without departing from the scope of the present invention.

The invention is explained further by means of the following figures and examples, whereby
- Fig. 1: is a diagrammatic representation of step c) of the method according to the invention, in which the binding molecules are incubated with the sample mixture;
- Fig. 2a: is a diagrammatic representation of one embodiment of step d) of the method according to the invention;
- Fig. 2b: is a diagrammatic representation of a further embodiment of step d);
- Fig. 2c: is a diagrammatic representation of a further embodiment of step d);

The "dividing up" of a sextuplet epitope into two triplet epitopes - preferably one for the C-terminal and one for the N-terminal end - in this case leads to a drastic reduction in the binding molecules necessary for the analysis of all possible peptides. In the present case, only 2 × 20³ - instead of 20⁶ for a sextuplet epitope - different binding molecules are required in order to be able to detect all possible peptides. Accordingly, in the claimed approach only 2 × 8000 antibodies are necessary, that is to say in each case 8000 for the N-terminal and 8000 for the C-terminal end of the peptides. It is thus possible by providing a library of 2 × 8000 binding molecules to detect any desired analyte peptide from a peptide mixture. By comparison therewith, with a sextuplet epitope more than 10⁷ binding molecules would be necessary - for an identical analysis.

Thus, contrary to the method described in WO 2004/031730 mentioned at the outset, the inventive concept resides in using two per se unspecific binding molecules that bind simultaneously to the analyte protein or analyte peptide and by this enable a specific enrichment and/or determination of the analyte protein/peptide.

A further advantage of the method according to the invention is that with the 2 × 20³ binding molecules it is possible to detect all N/C termini theoretically conceivable for proteinogenic amino acids of all peptides of any proteomes, independent of species.

It is understood that the selection of the amino acid triplets and thus of the amino acids depends per se on the sample to be investigated. Thus, depending on the sample to be investigated, account must also be taken of modified amino acids, or else - in the case of non-human samples - amino acids which are to be found only in animal, plant or microbial samples. It would be clear to the skilled person in this connection - based on the prior art available concerning this - which amino acids must be taken into account for which sample analysis.

The approach according to the invention thus makes possible for the first time an array-based proteome analysis.

In another embodiment of the method according to the invention, it is preferred for the detection and/or enrichment to take place with the use of second binding molecules which specifically recognize the analyte proteins/analyte peptides which are bound to the first binding molecules, with the second binding molecules being specific for a peptide-internal epitope.

It is advantageous in this embodiment that it is possible by the "preselection" of a limited multiplicity of analyte peptides/proteins by the first binding molecule for the complexity of the sample to be reduced, and the subsequent unambiguous detection takes place by means of the second binding molecule via the protein/peptide-specific internal epitope, so that detection is possible for exactly one targeted protein or peptide in a complex mixture with a reduced sample background.

In this connection, it is preferred in another embodiment for the second binding molecule to be specific for a peptide-internal or a terminal epitope, the epitopes having at least six amino acids.

It is advantageous in this embodiment that individual peptides can be identified in a very targeted manner through the use of second binding molecules with a high specificity.

It is generally preferred in the method according to the invention for the first and the second binding molecules to be selected from the group including antibodies, antibody fragments, aptamers, recombinant binding molecules.

The present disclosure further relates to binding molecules which are specific for the terminal peptide epitope of various peptide analytes, wherby the terminal binding molecule includes the free NH₂ group or the free COOH group, one or more than one amino acid defined by the protease specificity and up to a maximum of three further terminal amino acids and, where appropriate, additionally group-specific recognition sites.

It is preferred in this connection for the binding molecule to be selected from the group including antibodies, antibody fragments, aptamers, recombinant binding molecules.

The present disclosure further relates to the use of the binding molecule in a method according to the present invention.

The present disclosure further relates to a method for preparing binding molecules to be employed in the method according to the invention, in which peptide epitopes which are bound to a support and have a maximum of five amino acids are employed for immunization, selection and affinity maturation methods.

It is preferred in this connection for preparing the binding molecules to provide in a first step peptides having C- and N-terminal triplets, with the amino acid triplets displaying all possible amino acid combinations which are possible starting from 20 proteinogenic amino acids, and in the subsequent step to employ these peptides for immunization, selection and affinity maturation methods.

It is possible in this connection to employ for example classical immunization methods which are sufficiently well described in the state of the art (see, for example, Antibodies: A Laboratory Manual, by Ed Harlow, David Lane). On the other hand, the binding molecules can also be generated *in vitro,* in which case the binding pocket is constructed in such a way that the terminal NH₃⁺/COO⁻ function can bind optimally, for example in the form of a recess with appropriate opposite charge.

The methods employed for the synthetic preparation of the peptides employed for the immunization are likewise sufficiently well known in the state of the art (see, for example, Fmoc Solid Phase Peptide Synthesis, A Practical Approach by W.C. Chan and P.D. White (Eds), Oxford University Press).

Before incubating the sample mixture of step a) with the first binding molecule, a third binding molecule being specific for a peptide-internal epitope may be incubated with the sample mixture.

In this connection, it is preferred for the peptide-internal epitopes having at least six amino acids.

Thus, the third binding molecule serves like the second binding molecule in some embodiments as capture molecule whereas the first binding molecule now is used as some sort of universal detector molecule that preferably is labeled.

It is understood that the features and advantages mentioned above and to be explained hereinafter can be used not only in the stated combination but also alone or in other combinations without departing from the scope of the present invention.

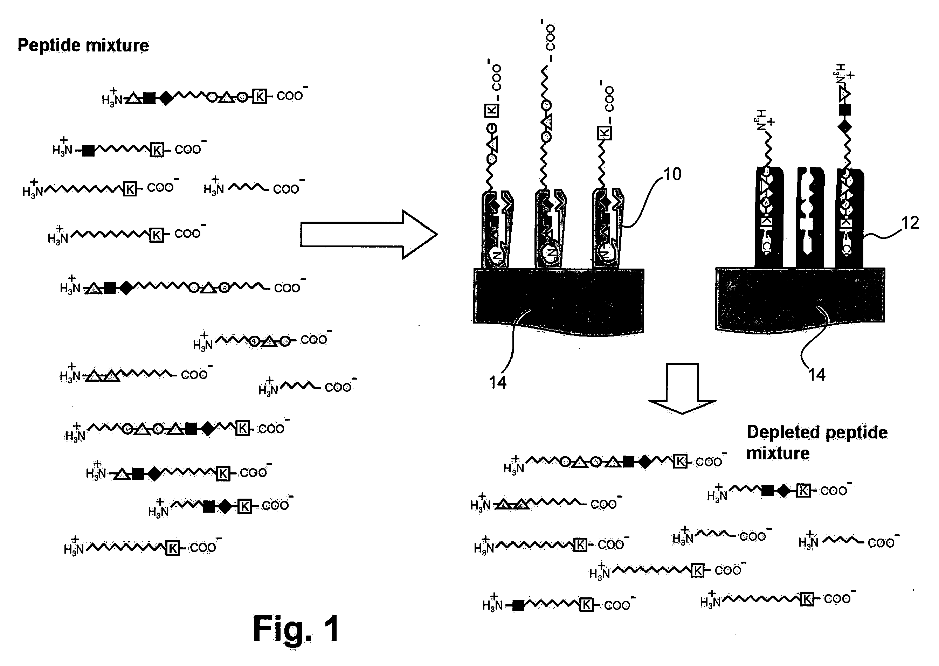

The invention is explained further by means of the following figures and examples, whereby
- Fig. 1: is a diagrammatic representation of step c) of the method according to the invention, in which the binding molecules are incubated with the sample mixture;

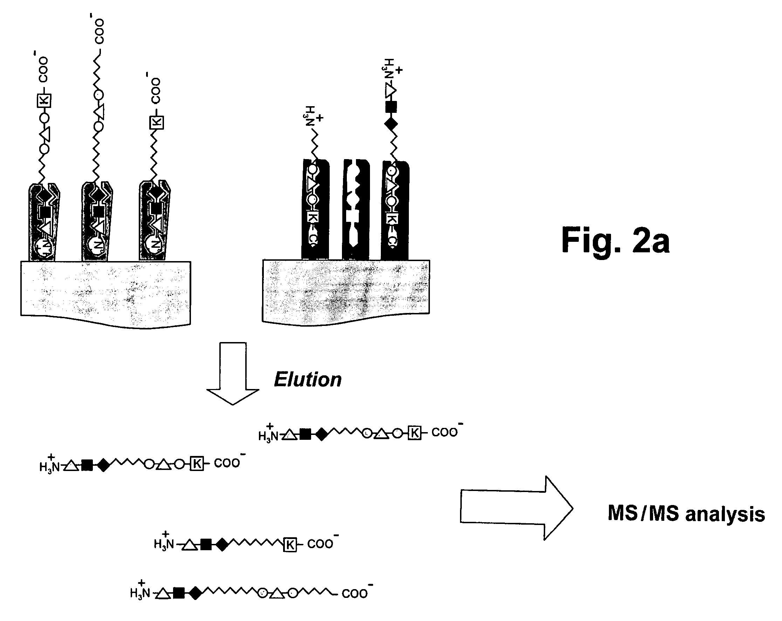

- Fig. 2a: is a diagrammatic representation of one embodiment of step d) of the method according to the invention;

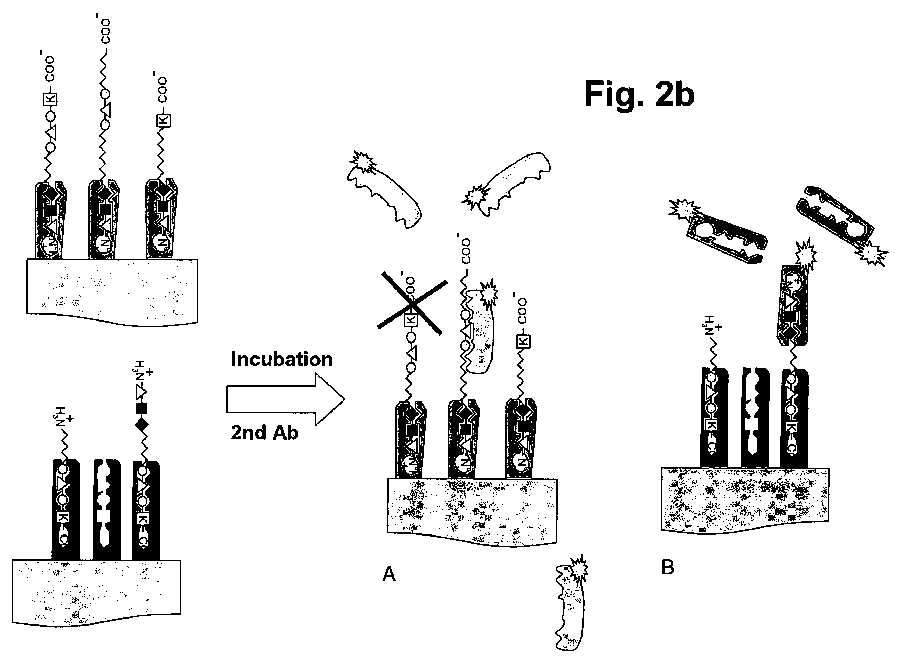

- Fig. 2b: is a diagrammatic representation of a further embodiment of step d);

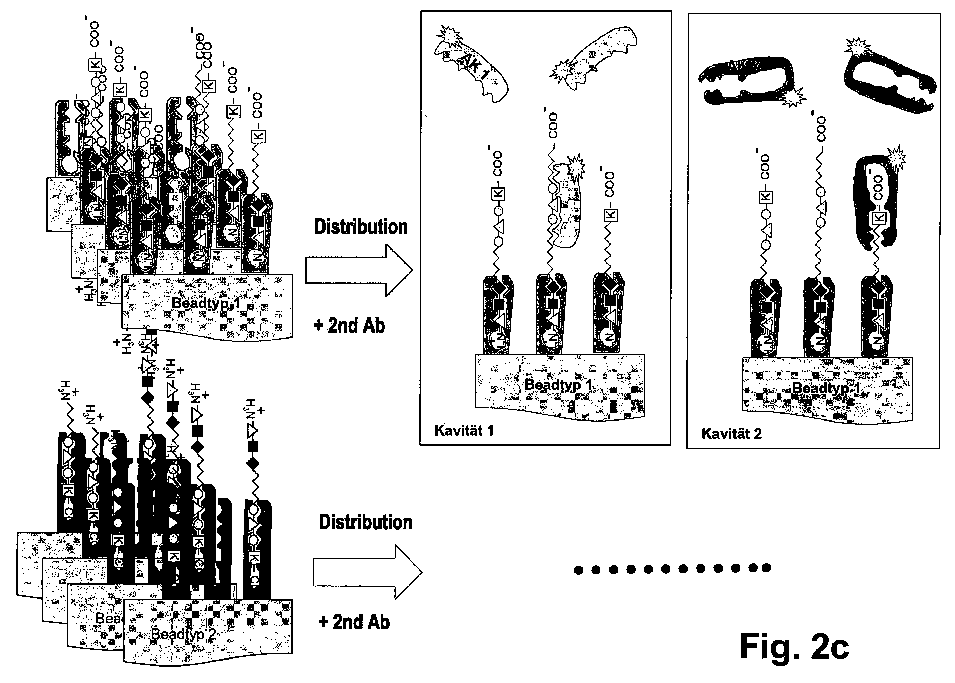

- Fig. 2c: is a diagrammatic representation of a further embodiment of step d);

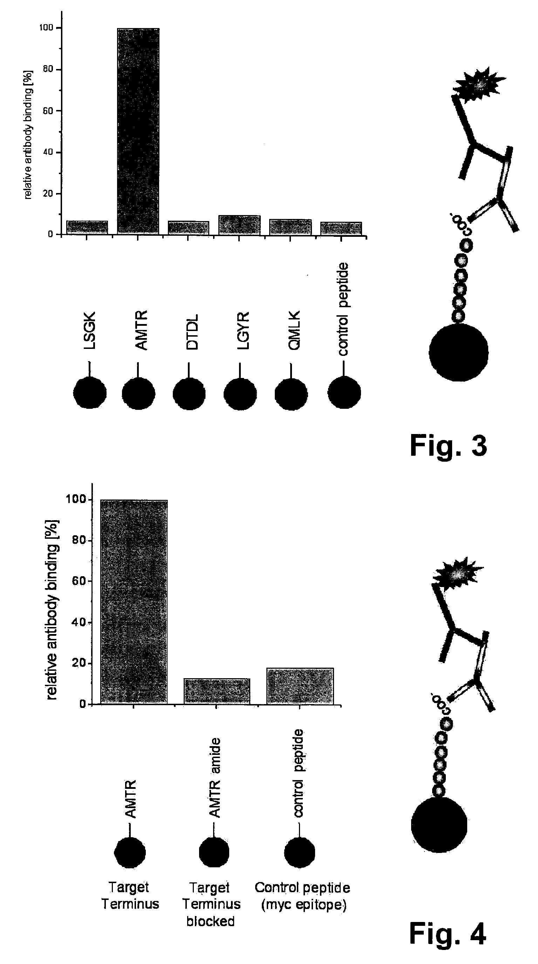

- Fig. 3: shows the determination of the cross-reactivity of a terminus specific antibody (AMTR) to other termini specific antibodies;
- Fig. 4: shows the determination of the terminus specificity of the antibody if fig. 3;

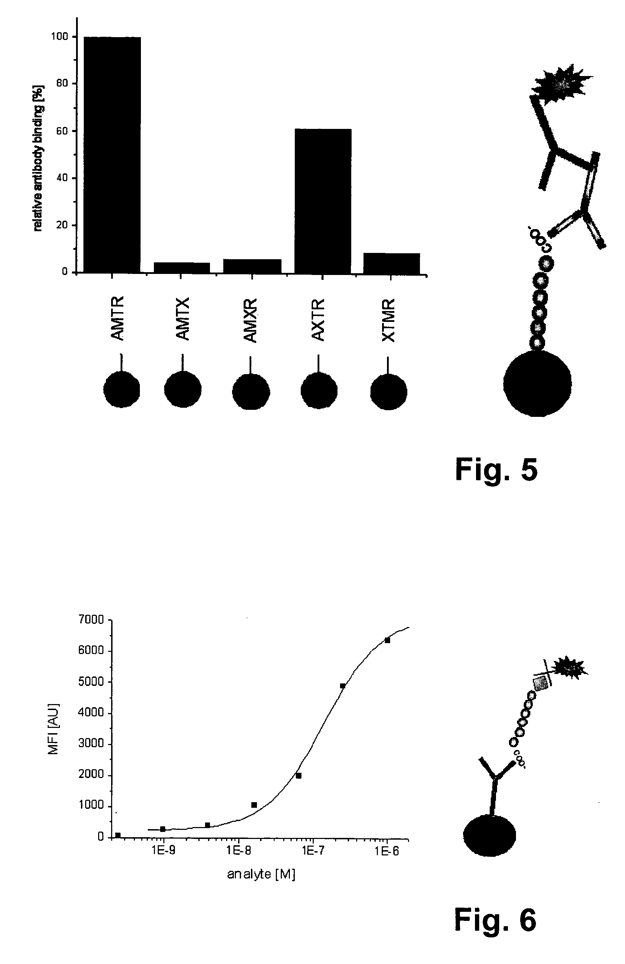

- Fig. 5: shows a X-positional peptide library scan for the antibody if fig. 3; and
- Fig. 6: shows the results of an immunocapture assay for the antibody if fig. 3;

Fig. 1 is a diagrammatic representation of the binding of the analyte peptides present in a sample mixture. The left-hand side of Fig. 1 depicts the sample mixture which has previously been treated with a specific protease so that (oligo)peptides are present in the mixture. The N-terminal end of the peptide is in this case designated H₃N⁺ and the C-terminal end COO⁻.

The right hand side of the diagrammatic representation shows how the various peptides are bound to the first binding molecules immobilized on a support. The binding molecules are in this case indicated by reference numbers 10 and 12, and the support by 14.

Thus, with reference to the method according to the invention and Fig. 1, provision of the sample mixture and of the first binding molecules is followed by incubation of these two together, whereby some of the peptides present in the sample mixture bind to the binding molecules. Unbound sample material is washed away.

Figures 1 and 2a to 2c are in each case a diagrammatic representation merely by way of example of the fact that the first binding molecules employed are specific for an epitope which includes the free NH₂ group or the free COOH group and in each case three amino acids. The exemplary embodiments shown in Figures 1 and 2a to 2d of the method according to the invention are merely by way of example, and many other exemplary embodiments are conceivable within the scope of the present invention; in particular, the binding molecules and the epitope to be recognized by the binding molecules can be configured otherwise.

Fig. 2a shows a diagrammatic representation of one embodiment of the method according to the invention with reference to step d), the detection of the bound analyte peptides. In this case, the analyte peptides bound to the binding molecules are eluted and then subjected to mass spectroscopy. Peptide subpopulations are in this case analysed by means of HPLC-MS/MS, with unambiguous identification taking place in sequence databases by combinatorial evaluation via sequence tag + peptide mass + partial epitope affinity fractionation + protease specificity.

In another embodiment of the method according to the invention, the binding molecules are immobilized on arrays of affinity matrices, for example affinity chips. Incubation with the sample mixture leads to binding of analyte peptides or peptide subpopulations to arrays of different affinity matrices. In the subsequent detection step, each point of the affinity array is examined by direct MALDI-based mass spectrometric analysis (SELDI).

Fig. 2b shows a diagrammatic representation of a further embodiment of the method according to the invention with reference to step d). In this case, the binding molecules were bound to a support; after incubation with the sample mixture, analyte peptides bind with one of their terminal ends to the binding molecules. The peptide subpopulations were then incubated with second binding molecules, so that the second binding molecules bind to the analyte peptides (see right-hand side of Fig. 2b, A and B). Depending on the specificity of the second binding molecules it is possible for example to achieve unambiguous identification by specific binding molecules which are specific for a peptide-internal epitope (six to seven amino acids) (see right-hand side of Fig. 2b, "A"). An ambiguous identification can also be achieved on the other hand by binding molecules which are specifically directed against the other terminal epitope of the analyte peptides. This is depicted in Fig. 2b, right-hand side, "B". The binding molecule can in this case - as shown in Fig. 2b - likewise be specific for an epitope which includes in each case the other free NH₂ group or COOH group and in each case three amino acids. This results as it were in a "split specific epitope" totalling 6 amino acids (3 amino acids relating to the first binding molecule + three amino acids relating to the second binding molecule). The specificity derives in this case from the combined binding specificity of the two binding molecules.

Finally, Fig. 2c shows a further embodiment of the method according to the invention in relation to step d): in the left-hand side of Fig. 2c, peptide subpopulations are bound via the first binding molecules to beads. These are then distributed over various cavities and incubated there with different second binding molecules. The second binding molecules may now - in analogy to Fig. 2b - in turn be specific for a peptide-internal epitope or else for the other terminal epitope which in turn includes the free NH₂ group or the free COOH group and three amino acids. Once again, the analyte peptides can be identified specifically by combinatorial use of two nonspecific binding molecules.

### 1. Example: Characterization of the monoclonal antibody 3D5 as selective for three carboxyl-terminal amino acids

The commercially available antibody anti-C-term His tag antibody 3D5 (Invitrogen, Carlsbad, CA) was investigated for its binding selectivity. This antibody was generated by immunizing a mouse with a fusion protein which has six histidines at the C terminus (see Lindner et al., "Specific detection of his-tagged proteins with recombinant anti-His tag scFv-phosphatase or scFv-phage fusions", Biotechniques 22, 140-149 (1997)). The epitope recognized by the antibody, and the selectivity of the binding for individual amino acid residues was investigated using a peptide array. Peptide libraries which represent variants of the terminal hexahistidine peptide were immobilized in directed fashion on microspheres (see Poetz at al., "Protein microarrays for antibody profiling: Specificity and affinity determination on a chip", Proteomics 5, 2402-2411 (2005)). This entails use, for each of the six terminal histidines, of a peptide position library in which, instead of the defined amino acids, mixtures of all 20 possible amino acids occur. It was possible to investigate the influence of the carboxyl terminus on the binding using a peptide which has the complete hexahistidine sequence but whose end has an amidated C terminus instead of a free COOH group (see Table 1).

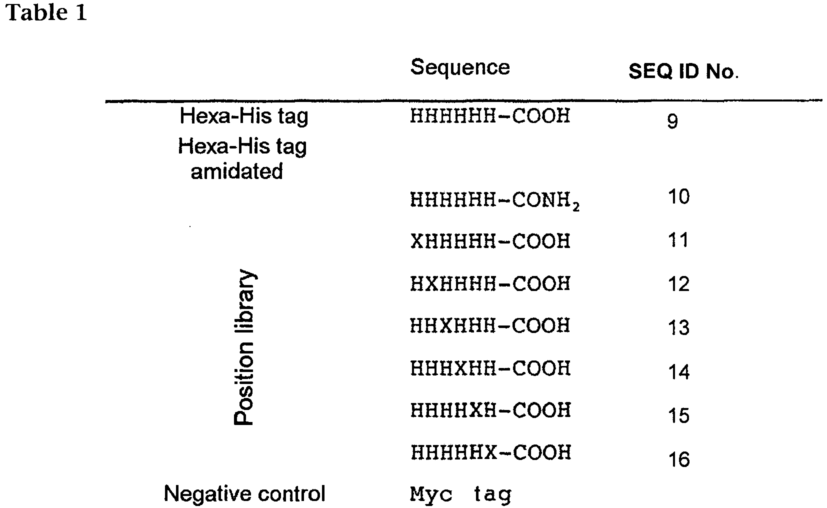

The peptides in Table 1 were synthesized as biotinylated peptides and immobilized on microspheres coated with N-avidin. For the binding studies, the microspheres were mixed with antibodies, and the binding of the antibody to the peptide was detected with the aid of a phycoerythrin-conjugated anti-mouse IgG and selected with a Luminex L100 (Austin, TX, USA). Randomization of the histidines at position 1, 2 , and 3 (starting from the C terminus, i.e. peptides having SEQ ID-No. 16, 15, 14, respectively) led to the decline in the measured signal, showing that these amino acids are necessary for the binding. The same applies to blocking of the free carboxyl group by amidation (peptide having SEQ ID-No. 10); this modification reduces the binding of the antibody to less than 15%, i.e. the negative charge of the free carboxyl group is obligatory for reaction of the antibody with its antigen. Replacement of the fourth, fifth and sixth histidine (peptides having SEQ ID-No. 13, 12, 11, respectively) by a mixture of all twenty amino acids led to no change in the binding. The recognized epitope of the described antibody therefore consists of 3 terminal amino acids and the free terminus.

The results of the selectivity assays are detailed in diagram 1 below.

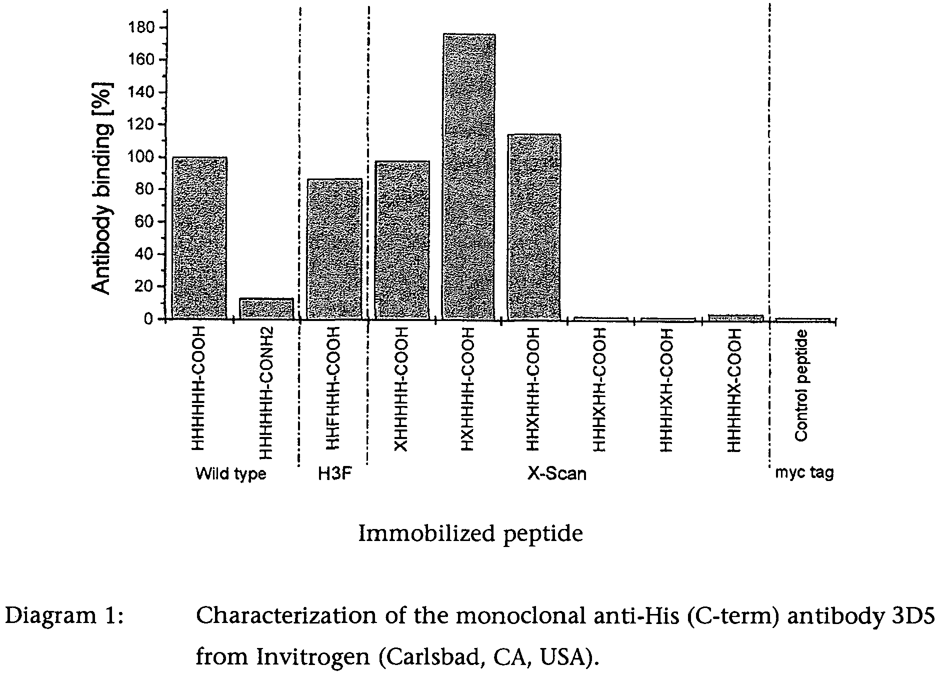

Thus, surprisingly, the antibody showed only selectivity for the three C-terminal histidines. Replacement of the subsequent histidines by the X position had scarcely any or no effect on the binding of the antibody. Furthermore, blocking of the negative charge of the C terminus by amidation likewise prevents binding of the antibody. The crystal structure of an scFv which was obtained from this antibody and has a hexahistidine peptide confirms this result (see Kaufmann et al., "Crystal structure of the anti-His tag antibody 3D5 single-chain fragment complexed to its antigen", J Mol Biol 318, 135-147 (2002)). The antibody binds to the backbone of the four C-terminal histidines, to the side chains of the three C-terminal histidines and to the carboxyl group of the terminal histidine. On the basis of these peptide array analyses and of the crystal structure, this commercial antibody can be referred to as a C-terminal tripeptide-specific antibody.

### 2. Example

On the basis of the results of the characterization of the antibody 3D5, various peptides with three histidines at the C and N terminus in combination with a peptide epitope were synthesized. In addition, the peptides with C-terminal histidine labelling were labelled at the N terminus with a glycine, and the peptides with N-terminal histidine labelling were labelled at the C terminus with serine, in order to be able to differentiate corresponding peptides from one another by mass spectrometry (see Table 2).

**Table 2**

| Peptide sequence | Peptide epitope | SEQ ID NO | MW [Da] |
|---|---|---|---|
| HHHGSGEQKLISEEDLG | c-myc | 1 | 1871.88 |
| HHHGSGYPYDVPDYAG | HA | 2 | 1770.74 |
| HHHGSGYTDIEMNRLGKG | HAV | 3 | 2007.93 |
| HHHGSGGKPIPNPLLGLDSTG | V5 | 4 | 2090.07 |
| SEQKLISEEDLGSGHHH | c-myc | 5 | 1901.89 |
| SYPYDVPDYAGSGHHH | HA | 6 | 1800.75 |
| SYTDIEMNRLGKGSGHHH | HAV | 7 | 2037.94 |
| SGKPIPNPLLGLDSTGSGHHH | V5 | 8 | 2120.08 |

### Immunoassay detection

The antibody and the antibodies specific for the peptide epitopes (see Table 2) were immobilized on microspheres by standard protocol. The peptides described above were added individually in various concentrations to serum. The peptides were detected firstly via the peptide epitope-specific antibodies and secondly via the His tag antibody.

### Detection by mass spectrometry

The His tag antibody 3D5 was chemically immobilized on carboxymethylcellulose. This material served as affinity matrix in order to purify the abovementioned peptides from a complex mixture. Only the peptides with C-terminal His tag and a free carboxyl group were subsequently detectable in the mass spectrometer.

### 3. Example I

### Experiment with β-catenin in silico digestion

The Wnt signalling pathway is very important during embryonic development in all animal species. Abnormal activation of this signalling pathway leads to tumorigene-sis. Mutations in the adenomatous polyposis coli (APC) or β-catenin protein result in nuclear accumulation of the β-catenin protein. In a complex with T-cell factor/lymphoid enhancing factor (TCF/LEF) β-catenin activates transcription factor genes which positively influence cell proliferation and thus promote uncontrolled cell growth.

Thus, β-catenin represents a classical proto-oncogene. Advantages of this protein as model protein are its relevance to oncology and its highly conserved sequence between different species. The human sequence and the classical model organisms (mouse, rat) are identical apart from one amino acid.

### In silico digestion

The β-catenin protein was digested *in silico* with trypsin with the aid of an EDP program (http://www.expasy.org/tools/peptidecutter/). The fragments were arranged according to length (see Table 3).

**Table 3: List of peptide fragments generated by an in silico digestion of β-catenin with trypsin. The fragments have been arranged according to peptide length.**

| **Position of the cleavage site** | **Name of the enzyme** | **Resulting peptide sequence** | **Peptide length [aa]** | **Peptide mass [Da]** | **SEQ ID No** |
|---|---|---|---|---|---|
| 19 | Trypsin | K | 1 | 146.19 | |
| 181 | Trypsin | K | 1 | 146.19 | |
| 672 | Trypsin | K | 1 | 146.19 | |
| 550 | Trypsin | R | 1 | 174.20 | |
| 673 | Trypsin | R | 1 | 174.20 | |
| 435 | Trypsin | NK | 2 | 260.29 | |
| 95 | Trypsin | VR | 2 | 273.34 | |
| 93 | Trypsin | AQR | 3 | 373.41 | |
| 345 | Trypsin | VLK | 3 | 358.48 | |
| 457 | Trypsin | AGDR | 4 | 417.42 | 17 |
| 185 | Trypsin | EASR | 4 | 461.48 | 18 |
| 591 | Trypsin | IVIR | 4 | 499.65 | 19 |
| 274 | Trypsin | MAVR | 4 | 475.61 | 20 |
| 292 | Trypsin | TNVK | 4 | 460.53 | 21 |
| 453 | Trypsin | TVLR | 4 | 487.60 | 22 |
| 587 | Trypsin | DVHNR | 5 | 639.67 | 23 |
| 190 | Trypsin | HAIMR | 5 | 626.78 | 24 |
| 474 | Trypsin | HLTSR | 5 | 612.69 | 25 |
| 666 | Trypsin | MSEDK | 5 | 608.66 | 26 |
| 671 | Trypsin | PQDYK | 5 | 649.70 | 27 |
| 335 | Trypsin | TYTYEK | 6 | 803.87 | 28 |
| 549 | Trypsin | AHQDTQR | 7 | 854.88 | 29 |
| 158 | Trypsin | AIPELTK | 7 | 770.92 | 30 |
| 515 | Trypsin | ATVGLIR | 7 | 728.89 | 31 |
| 535 | Trypsin | EQGAIPR | 7 | 769.86 | 32 |
| 281 | Trypsin | LAGGLQK | 7 | 685.82 | 33 |
| 342 | Trypsin | LLWTTSR | 7 | 876.02 | 34 |
| 542 | Trypsin | LVQLLVR | 7 | 840.08 | 35 |
| 288 | Trypsin | MVALLNK | 7 | 788.02 | 36 |
| 717 | Trypsin | QDDPSYR | 7 | 879.88 | 37 |
| 233 | Trypsin | EGLLAIFK | 8 | 890.09 | 38 |
| 394 | Trypsin | NLSDAATK | 8 | 818.88 | 39 |
| 133 | Trypsin | LAEPSQMLK | 9 | 1016.22 | 40 |
| 242 | Trypsin | SGGIPALVK | 9 | 841.02 | 41 |
| 354 | Trypsin | VLSVCSSNK | 9 | 936.09 | 42 |
| 180 | Trypsin | AAVMVHQLSK | 10 | 1083.31 | 43 |
| 496 | Trypsin | LHYGLPWVK | 10 | 1124.39 | 44 |
| 386 | Trypsin | LVQNCLWTLR | 10 | 1245.51 | 45 |
| 200 | Trypsin | SPQMVSAIVR | 10 | 1087.30 | 46 |
| 684 | Trypsin | LSVELTSSLFR | 11 | 1251.45 | 47 |
| 469 | Trypsin | EDITEPAICALR | 12 | 1330.52 | 48 |
| 486 | Trypsin | HQEAEMAQNAVR | 12 | 1383.50 | 49 |
| 508 | Trypsin | LLHPPSHWPLIK | 12 | 1437.75 | 50 |
| 170 | Trypsin | LLNDEDQVVVNK | 12 | 1385.54 | 51 |
| 212 | Trypsin | TMQNTNDVETAR | 12 | 1379.46 | 51 |
| 225 | Trypsin | CTAGTLHNLSHHR | 13 | 1446.61 | 53 |
| 528 | Trypsin | NLALCPANHAPLR | 13 | 1389.64 | 54 |
| 625 | Trypsin | VAAGVLCELAQDK | 13 | 1316.54 | 55 |
| 449 | Trypsin | MMVCQVGGIEALVR | 14 | 1505.87 | 56 |
| 661 | Trypsin | NEGVATYAAAVLFR | 14 | 1481.67 | 57 |
| 565 | Trypsin | TSMGGTQQQFVEGVR | 15 | 1624.79 | 58 |
| 329 | Trypsin | LIILASGGPQALVNIMR | 17 | 1766.18 | 59 |
| 582 | Trypsin | MEEIVEGCTGALHILAR | 17 | 1842.16 | 60 |
| 151 | Trypsin | HAVVNLINYQDDAELATR | 18 | 2042.24 | 61 |
| 18 | Trypsin | MATQADLMELDMAMEPDR | 18 | 2068.38 | 62 |
| 312 | Trypsin | FLAlTTDCLQILAYGNQESK | 20 | 2228.55 | 63 |
| 612 | Trypsin | GLNTIPLFVQLLYSPIENIQR | 21 | 2428.86 | 64 |
| 647 | Trypsin | EAAEAIEAEGATAPLTELLHSR | 22 | 2279.49 | 65 |
| 376 | Trypsin | PAIVEAGGMQALGLHLTDPSQR | 22 | 2261.58 | 66 |
| 710 | Trypsin | TEPMAWNETADLGLDIGAQGEPLGYR | 26 | 2805.07 | 67 |
| 270 | Trypsin | MLGSPVDSVLFYAITTLHNLLLHQEGAK | 28 | 3068.58 | 68 |
| 124 | Trypsin | AAMFPETLDEGMQIPSTQFDAAHPTNVQR | 29 | 3203.55 | 69 |
| 49 | Trypsin | AAVSHWQQQSYLDSGIHSGATTTAPSLSGK | 30 | 3086.32 | 70 |
| 433 | Trypsin | QEGMEGLLGTLVQLLGSDDINWTCAAGILSNLTCNNYK | 39 | 4068.64 | 71 |
| 90 | Trypsin | GNPEEEDVDTSQVLYEWEQGFSQSFTQEQVADIDGQYAMTR | 41 | 4728.95 | 72 |
| 781 | End of the sequence | | 64 | 6822.39 | 73 |

### Selection of the termini with subsequent database search

The fragments and the termini were examined from various points of view. It was firstly intended that the fragments have a length of 20 amino acids or more in order to make construction of a sandwich immunoassay possible. A shorter fragment length does not appear sensible for steric reasons, because otherwise the two epitopes of the peptide antigen are where appropriate not simultaneously available for binding by the first and second binding molecules (capture and detection antibodies) in an assay.

Because of the structural properties of the protein it was additionally advantageous to select fragments near the N or C terminus. Both N terminus and C terminus are, on the basis of investigations of the crystal structure and other methods (see Huber et al., Cell 1997, 90, 871-882), readily accessible to proteolysis. This makes it possible to generate target peptides with a proteolytic digestion without denaturing conditions.

The fragment bcat_TTF1 (SEQ ID No. 70) was selected because the mutations responsible for the development of a tumour occur in this region.

The fragment bcat_TTF1 (SEQ ID No. 70) is not a tryptic fragment but is a fragment which would be produced by digestion with the endoproteinase LysC. The termini of this fragmentary piece were selected in order that a further enzyme can also be used alternatively for the digestion.

| Fragment Code | Target | Fragment | Length | Cleavage position | SEQ ID No. |
|---|---|---|---|---|---|
| bcat_TTF1 | Ctnnb1_1 | AAVSHWQQQSYLDSGIHSGATTTAPSLSGK | 30 | 49 | 70 |
| bcat_TTF2 | Ctnnb1_2 | | 41 | 90 | 72 |
| bcat_TTF3 | Ctnnb1_3 | | 64 | End of the sequence | 73 |
| bcat_TTF4 | Ctnnb1_4 | TEPMAWNETADLGLDIGAQGEPLGYR | 26 | 710 | 67 |
| bcat_TLCF1 | Ctnnb1_5 | | 84 | 133 | 74 |

### Database search for fragments

A human, non-redundant protein database was searched for termini-specific sequences (three amino acids of N terminus and four amino acids of C terminus) of the selected peptide fragments. The results represent all potential N and C termini which might be produced by tryptic digestion of the human proteome. These subproteomes can be analyzed after affinity purification by the generated termini-specific antibodies for example using mass spectrometry.

The database search was additionally restricted by searching simultaneously for both termini. In the search, 100 amino acids were allowed without restriction between the two termini. The database was thus searched for trypsin fragments with a length of up to 107 amino acids and the respective specific termini. However, in the combination search it was not possible, because of the software, to preclude internal trypsin cleavage sites in the output fragments. After eliminating internal trypsin cleavage sites from the hits of the combination search it was possible to reduce the number of proteins found to one hit, namely the target protein β-catenin, i.e. two termini-specific antibodies respectively for three and for four amino acids are sufficient to achieve a 100% hit rate for the target protein in all cases considered.

The results of the search are summarized in the table below (Table 4).

**Table 4: Database search for number of proteins which contain appropriate termini after tryptic digestion**

| NPIR database hits | | | | | | | | |
|---|---|---|---|---|---|---|---|---|
| N Terminus | | C Terminus | | * | | Combination after elimination | | * |
| 3740 | [RK]AAV | 1219 | LSGK(P) | 75 | 8 | 1 | [RK]AAVX(1,100)LSGK{P} | 80 |
| 797 | [RK]GNP | 190 | AMTR(P) | 76 | 2 | 1 | [RK]GNPX(1,100)AMTR{P} | 81 |
| 471 | [RK]SFH | 2 | DTDL> | 77 | 2 | 1 | [RK]SFHX(1,100)DTDL> | 82 |
| 1101 | [RK]TEP | 418 | LGYR(P) | 78 | 2 | 1 | [RK]TEPX(1,100)LGYR{P} | 83 |
| 797 | [RK]GNP | 331 | QMLK(P) | 79 | 2 | 1 | [RK]GNPX(1,100)QMLK{P} | 84 |

| | | | | | | | | |
|---|---|---|---|---|---|---|---|---|
| *SEQ ID No. | | | | | | | | |

### 4. Example II

### Experiment with β-catenin in vitro

The fragment termini identified from the *in silico* digest of ß-Catenin were used for the generation of terminus specific antibodies, binding to the last 3 or 4 amino acid and the terminus of the peptide. These antibodies have been characterized by incubation with peptide arrays.

Table 5 shows successful immunizations.

**Table 5: Successful immunizations**

| **N-termini** | **C-termini** | **SEQ ID No.** | **Rabbit polylonal** | **Rat monoclonal** |
|---|---|---|---|---|
| **AAV-** | | | | **successful** |
| **TEP-** | | | | **successful** |
| | **-LSGK** | 75 | **successful** | |
| | **-AMTR** | 76 | **successful** | **successful** |
| | **-DTDL** | 77 | **successful** | **successful** |
| | **-LGYR** | 78 | **successful** | **successful** |
| | **-QMLK** | 79 | **successful** | |
| | **-APFK** | 85 | | **successful** |

### Immunization strategy

The fragment termini shown in table 4 (the last three or four amino acids of the C-or N-terminal end of a tryptic peptide fragment) were synthesized using standard peptide chemistry. Three spacers (8-amino-3,6-dioxaoctanoic acid, DOA) were added to the targeted three or four amino acids antigen. At the non targeted terminus of the peptide a cystein group was added (e.g. C-Doa-Doa-Doa-AMTR; SEQ ID No. 86). The thiol-group of the cystein allowed an oriented conjugation via a bifunctional linker (e.g. m-Maleimidobenzoyl-N-hydroxysulfosuccinimide ester, sulfo-MBS) to a carrier protein (e.g. keyhole limpet hemocyanine or ovalbumin). The peptide carrier protein conjugates were used to carry out standard immunization protocols in rabbits and rats to generate polyclonal and monoclonal antibodies.

### Characterisation of terminus specific antibodies

The generated antisera or monoclonal antibodies were tested for specificity and cross-reactivity using peptide arrays. Each array consisted of peptides containing the immunogens (free terminus and 3 or 4 amino acids and spacer), the target sequences fused to myc and ha peptides. In addition, the target sequences fused to other peptides were blocked at the N- or C-terminus, using acetylation or amidation, respectively.

These peptides allowed to analyze the influence/contribution of the free terminus for the binding of its appropriate antibody. Furthermore, a set of different peptide libraries was synthesized. Each specific amino acid position of the target terminus was randomized allowing the presence of one out of all of the 20 amino acids. These X-positional-library peptides provide information about the influence of each individual position on the antigen-antibody binding. A dramatic loss of binding signal reveals whether this position contributes strongly to the antigen-antibody interaction, no loss of binding signal reveals, that this position does not contribute significantly to the antigen-antibody interaction.

**Table 6: Sequences of the peptides for one target terminus (AMTR)**

| Description | Peptides on peptide array | SEQ ID No |
|---|---|---|
| Immunogen | CDoaDoaDoa**AMTR** | 86 |
| Target terminus on different peptides | CEQKLISEEDL**AMTR** | 87 |
| | CYPYDVPDYA**AMTR** | 88 |
| Target terminus blocked | CEQKLISEEDL**AMTR** synthesized as amide | 89 |
| | CYPYDVPDYA**AMTR** synthesized as amide | 90 |
| X-positional-peptide-library | CSEEDL**AMTX** | 91 |
| | CSEEDL**AMXR** | 92 |
| | CSEEDL**AXMR** | 93 |
| | CSEEDL**XMTR** | 94 |

All antisera and antibodies were incubated with the described peptide arrays. The results allowed the evaluation of the immunization process, determination of cross-reactivity to other termini sequences, determination of the influence of the spacer on the antibody-antigen reaction, determination of the specificity of the antibody specific to a free terminus, and influence of individual amino acid position to the antigen - antibody binding

Figure 3 shows the determination of the cross-reactivity. The target termini were synthesized and immobilised on microspheres. The target terminus specific polyclonal serum - here AMTR - was incubated with the different microspheres. Antibody binds only to its target terminus and not to the other target termini. This demonstrates, that this antibody is specific for its target terminus.

Figure 4 shows the determination of the terminus specificity. The target peptide was synthesized with a free carboxyl function and as a amide function at the immunogenic terminus. Peptides were immobilised on microspheres and incubated with a target terminus specific polyclonal serum. The antibody does not bind to the amide version of the target terminus. This demonstrates, that the antibody is terminus specific and the free carboxy function is required for the antibody binding. The antibody binds only if the sequence, here AMTR occurs at the C-terminus of a peptide or a protein.

Figure 5 shows a X-positional peptide library scan. A set of different peptide libraries was synthesized, in which every amino acid of the target terminus AMTR was randomized by allowing the presence of all 20 amino acids. An array containing X-positional-library peptides provide information about the influence of the single amino acid residue on the antigen-antibody reaction. A dramatic loss of binding signal compared to the original epitope reveals whether this position contributes strongly to the antigen-antibody interaction. No loss of binding signal reveals that this position does not contribute significantly to the antigen-antibody interaction. For this antibody, the side chains of the amino acids A, T and R reveal strongest influence on the binding event.

### Purification

Polyclonal rabbit as well as monoclonal rat antibodies were purified according standard procedures with either peptide or Protein G affinity columns.

### Immunocapture assay

The capture capability of the purified antibodies was tested in a simple immunoassay set up. The targeted peptide fragment identified from the *in silico* digest was synthesized in a biotinylated form. The peptide was incubated in the presence of a complex peptide mixture - a 6 mer peptide library- with the antibody immobilised on a microsphere. The captured peptide was detected with a fluorescently labeled streptavidin.

Figure 6 shows the results of an immunocapture assay. The terminal specific antibody generated against the C-terminus AMTR was immobilised on a microsphere. Biotinylated peptide containing AMTR at the C-terminus was incubated in different concentrations with the immobilised antibody. Captured peptide was detected with fluorescently labeled Streptavidin. Specific peptide analyte could be detected in lower nanomolar range.

### Affinitiy mass spectrometry approach

The antibodies were tested in an affinity mass spectrometry experiment. The AMTR specific terminal antibody was immobilized on a column. A peptide containing AMTR at the C-terminus was mixed with 4 other different peptides and loaded on the affinity column. After elution with a glycine buffer pH 2.5, the flowthrough, the eluted fraction and the starting mixture (injected sample) was analysed with a mass spectrometer. The AMTR specifc target peptide was captured quantitatively out of the mixture and could be eluted using the glycine buffer. None of the other peptides was detectable in the eluted fraction from the anti-AMTR Ab affinity column. Furthermore the affinity capture of the AMTR Peptide on the anti-AMTR Ab column resulted in a more than 5-fold signal increase in HPLC-ESI-mass spectrometry.

**Table 7: List of peptides used for proof of concept study of the affinity mass spectrometry approach. Peptide labels, peptide sequences and the calculated monoisotopic peptide masses of the different possible protonated peptide signals in the ESI mass spectra are listed. Peptides were used as non purified, crude peptides. Therefore, unidentified contaminants from side reactions of peptide synthesis were part of the mixture.**

| **Peptide mixture** | | | | | | | |
|---|---|---|---|---|---|---|---|
| **No.** | **Sequence** | **Molecular Mass** | **Ion masses [Da] (calculated)** | | | | **SEQ ID No.** |
| | | **[Da]** | **[M+H]⁺** | **[M+2H]²⁺** | **[M+3H]³⁺** | **[M+4H]⁴⁺** | |
| 1: | DNP-DGGQY AMTR-OH | 1163.4 | 1164.4 | 582.7 | 388.8 | 291.9 | **95** |
| 2: | DNP-EQKLISEEDLHHH-OH | 1779.8 | 1780.8 | 890.9 | 594.3 | 446.0 | **96** |
| 3: | DNP-EQKLISEEDL-Doa-Doa-Doa-HHH-OH | 2115.0 | 2216.0 | 1108.5 | 739.3 | 554.8 | **97** |
| 4: | DNP-YPYDVPDYA-Doa-Doa-Doa-HHH-OH | 2113.9 | 2114.9 | 1057.9 | 705.6 | 529.5 | **98** |
| 5: | DNP-YTDIEMNRLGK-Doa-Doa-Doa-HHH-OH | 2351.1 | 2352.1 | 1176.5 | 784.7 | 588.8 | **99** |

| | | | | | | | |
|---|---|---|---|---|---|---|---|
| DNP = Dinitrophenyl- DOA = 3,6-Dioxa-8-aminooctanoic acid | | | | | | | |

## Claims

1. Method for the detection and/or enrichment of a large number of different analyte proteins and/or analyte peptides from a sample mixture which includes proteins and/or peptides, whereby the method includes the following steps:
a) provision of the sample mixture and fragmentation of the proteins contained therein into defined peptides by tryptic digestion,
b) provision of first binding molecules which are specific for a peptide epitope of a large number of the various analyte proteins and/or analyte peptides, whereby the peptide epitope includes three, four or five amino acids,
c) incubation of the first binding molecules with the sample mixture, and
d) detection and/or enrichment of the analyte proteins and/or analyte peptides bound to the first binding molecules, by using second binding molecules which are specific for a peptide epitope of a large number of the various analyte proteins and/or analyte peptides, whereby the peptide epitope includes three, four or five amino acids, and specifically recognize analyte proteins and/or analyte peptides which are bound to the first binding molecules, wherein
the first binding molecules are specific for one of the two terminal peptide epitopes of the analyte peptides, whereby the terminal peptide epitope includes the free NH₂ group or the free COOH group and three to five amino acids, and
the second binding molecules are specific for the other terminal peptide epitopes of the analyte peptides, whereby the terminal peptide epitope includes the free COOH group or the free NH₂ group and three to five amino acids,
such that an analyte peptide is detected by combined binding of first and second binding molecules to both free terminal epitopes of the analyte peptide.

2. Method according to claim 1, **characterized in that** a sample mixture with denatured analyte proteins and/or analyte peptides is provided in step a).

3. Method according to claims 1 or 2, **characterized in that** first binding molecules which display amino acid group-specific recognition at one or more positions of the peptide epitope are provided in step b).

4. Method according to any of claims 1 to 3, **characterized in that** the first binding molecules are immobilized on a support.

5. Method according to claim 4, **characterized in that** the support is selected from the group including microarrays, support material for affinity columns, chromatography materials, microchannel structures, capillary surfaces, sensor surfaces, polymeric porous sponge structures, beads.

6. Method according to any of claims 1 to 5, **characterized in that** the detection and/or enrichment in step d) takes place by simultaneous binding of two different binding molecules to different epitopes of the analyte protein and/or analyte peptide by methods such as FRET or proximity ligation assay.

7. Method according to any of claims 1 to 6, **characterized in that** the first and second binding molecules are incubated in solution with the sample.

8. Method according to any of claims 1 to 7, **characterized in that** antibodies, antibody fragments, aptamers, recombinant binding molecules are employed as first and/or second binding molecules.

## Patentansprüche

1. Verfahren zum Nachweisen und/oder Anreichern einer Vielzahl von verschiedenen Analytproteinen und/oder Analytpeptiden aus einer Probenmischung, die Proteine und/oder Peptide aufweist, wobei das Verfahren die folgenden Schritte aufweist:
a) Bereitstellen der Probenmischung und Fragmentierung der darin enthaltenen Proteine in definierte Peptide durch tryptischen Verdau;
b) Bereitstellen von ersten Bindemolekülen, die spezifisch für ein Peptidepitop einer Vielzahl der verschiedenen Analytproteine und/oder Analytpeptide sind, wobei die Peptidepitope drei, vier oder fünf Aminosäuren umfassen;
c) Inkubieren der ersten Bindemoleküle mit der Probenmischung; und
d) Nachweisen und/oder Anreichern der an die ersten Bindemoleküle gebundenen Analytproteine und/oder Analytpeptide, indem zweite Bindemoleküle verwendet werden, die spezifisch für ein Peptidepitop einer Vielzahl der verschiedenen Analytproteine und/oder Analytpeptide sind, wobei die Peptidepitope drei, vier oder fünf Aminosäuren umfassen, und spezifisch Analytproteine und/oder Analytpeptide erkennen, die an die ersten Bindemoleküle gebunden sind, wobei
die ersten Bindemoleküle spezifisch für eines der beiden terminalen Peptidepitope der Analytpeptide sind, wobei die terminalen Peptidepitope die freie NH₂-Gruppe oder die freie COOH-Gruppe und drei bis fünf Aminosäuren umfassen,
die zweiten Bindemoleküle spezifisch für die anderen terminalen Peptidepitope der Analytpeptide sind, wobei die terminalen Peptidepitope die freie COOH-Gruppe oder die freie NH₂-Gruppe und drei bis fünf Aminosäuren umfassen,
so dass die Analytpeptide durch kombiniertes Binden von ersten und zweiten Bindemoleküle an beide freien terminalen Peptidepotope nachgewiesen werden.

2. Verfahren nach Anspruch 1, **dadurch gekennzeichnet, dass** in Schritt a) eine Probenmischung mit denaturierten Analytproteinen und/oder Analytpeptiden bereitgestellt wird.

3. Verfahren nach Anspruch 1 oder 2, **dadurch gekennzeichnet, dass** in Schritt b) erste Bindemoleküle bereitgestellt werden, die an einer oder mehreren Positionen des Peptidepitops eine Aminosäurengruppen-spezifische Erkennung aufweisen.

4. Verfahren nach einem der Ansprüche 1 bis 3, **dadurch gekennzeichnet, dass** die ersten Bindemoleküle auf einem Träger immobilisiert sind.

5. Verfahren nach Anspruch 4, **dadurch gekennzeichnet, dass** der Träger ausgewählt ist aus der Gruppe umfassend Mikroarrays, Trägermaterial für Affinitätssäulen, Chromatographiematerialien, Mikrokanalstrukturen, Kapillaroberflächen, Sensoroberflächen, polymere poröse Schwammstrukturen, Beads.

6. Verfahren nach einem der Ansprüche 1 bis 5, **dadurch gekennzeichnet, dass** das Nachweisen und/oder Anreichern in Schritt d) durch gleichzeitige Bindung von zwei verschiedenen Bindemolekülen an verschiedenen Epitopen des Analytproteins und oder Analytpeptids durch Verfahren wie FRET oder proximity ligation assay erfolgt.

7. Verfahren nach einem der Ansprüche 1 bis 6, **dadurch gekennzeichnet, dass** die ersten und zweiten Bindemoleküle in Lösung mit der Probe inkubiert werden.

8. Verfahren nach einem der Ansprüche 1 bis 7, **dadurch gekennzeichnet, dass** als erste und/oder zweite Bindemoleküle Antikörper, Antikörperfragmente, Aptamere, rekombinante Bindemoleküle eingesetzt werden.

## Revendications

1. Procédé pour la détection et/ou l'enrichissement d'un grand nombre de protéines à analyser et/ou de peptides à analyser différent(e)s à partir d'un mélange d'échantillons qui comporte des protéines et/ou des peptides, où le procédé comporte les étapes suivantes :
a) la fourniture du mélange d'échantillons et la fragmentation des protéines contenues dedans en des peptides définis par digestion trypsique,
b) la fourniture de premières molécules de liaison qui sont spécifiques à un épitope peptidique d'un grand nombre des divers(es) protéines à analyser et/ou peptides à analyser, où l'épitope peptidique comporte trois, quatre ou cinq acides aminés,
c) l'incubation des premières molécules de liaison avec le mélange d'échantillons, et
d) la détection et/ou l'enrichissement des protéines à analyser et/ou des peptides à analyser lié(e)s aux premières molécules de liaison, en utilisant des deuxièmes molécules de liaison qui sont spécifiques à un épitope peptidique d'un grand nombre des divers(es) protéines à analyser et/ou peptides à analyser, où l'épitope peptidique comporte trois, quatre ou cinq acides aminés, et qui reconnaissent spécifiquement des protéines à analyser et/ou des peptides à analyser qui sont lié(e)s aux premières molécules de liaison, où
les premières molécules de liaison sont spécifiques à l'un des deux épitopes peptidiques terminaux des peptides à analyser, où l'épitope peptidique terminal comporte le groupe NH₂ libre ou le groupe COOH libre et trois à cinq acides aminés, et
les deuxièmes molécules de liaison sont spécifiques aux autres épitopes peptidiques terminaux des peptides à analyser, où l'épitope peptidique terminal comporte le groupe COOH libre ou le groupe NH₂ libre et trois à cinq acides aminés,
de sorte qu'un peptide à analyser soit détecté par liaison combinée de premières et deuxièmes molécules de liaison aux deux épitopes terminaux libres du peptide à analyser.

2. Procédé selon la revendication 1, **caractérisé en ce qu'**un mélange d'échantillons avec des protéines à analyser et/ou des peptides à analyser dénaturé(e)s est fourni à l'étape a).

3. Procédé selon la revendication 1 ou 2, **caractérisé en ce que** des premières molécules de liaison qui présentent une reconnaissance spécifique à un groupe d'acides aminés à une ou plusieurs position(s) de l'épitope peptidique sont fournies à l'étape b).

4. Procédé selon l'une des revendications 1 à 3, **caractérisé en ce que** les premières molécules de liaison sont immobilisées sur un support.

5. Procédé selon la revendication 4, **caractérisé en ce que** le support est choisi dans le groupe comportant des microréseaux, un matériau de support pour des colonnes d'affinité, des matériaux de chromatographie, des structures à microcanaux, des surfaces capillaires, des surfaces de détection, des structures spongieuses poreuses polymères, et des billes.

6. Procédé selon l'une des revendications 1 à 5, **caractérisé en ce que** la détection et/ou l'enrichissement à l'étape d) s'effectuent/s'effectue par liaison simultanée de deux molécules de liaison différentes à des épitopes différents de la protéine à analyser et/ou du peptide à analyser par des procédés tels qu'un procédé FRET ou le test de ligature de proximité.

7. Procédé selon l'une des revendications 1 à 6, **caractérisé en ce que** les premières et deuxièmes molécules de liaison sont incubées dans une solution avec l'échantillon.

8. Procédé selon l'une des revendications 1 à 7, **caractérisé en ce que** des anticorps, des fragments d'anticorps, des aptamères et des molécules de liaison recombinantes sont utilisés comme premières et/ou deuxièmes molécules de liaison.
